(19) Europäisches Patentamt
European Patent Office
Office européen des brevets

(11) EP 2 248 508 B1

(12) # FASCICULE DE BREVET EUROPEEN

(45) Date de publication et mention de la délivrance du brevet:
**20.04.2016 Bulletin 2016/16**

(21) Numéro de dépôt: **10160802.4**

(22) Date de dépôt: **22.04.2010**

(51) Int Cl.:
***A61K 8/06*** (2006.01) ***A61K 8/44*** (2006.01)
***A61K 8/60*** (2006.01) ***A61K 8/92*** (2006.01)
***A61Q 1/02*** (2006.01) ***A61Q 1/10*** (2006.01)

(54) **Emulsion cire-dans-eau comprenant l'association d'un derive d'acide glutamique et d'un alkylpolyglycoside**

Wachs-in-Wasser Emulsion, die eine Verbindung aus einem Glutaminsäurederivat und einem Alkylpolyglykosid enthält.

Wax-in-water emulsion including the combination of a glutamic acid derivative and an alkyl polyglycoside.

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **30.04.2009 FR 0952902**

(43) Date de publication de la demande:
**10.11.2010 Bulletin 2010/45**

(73) Titulaire: **L'Oréal**
**75008 Paris (FR)**

(72) Inventeurs:
- **Arditty, Stéphane**
**91160, Ballainvilliers (FR)**
- **Lahousse, Florence**
**94320, Thiais (FR)**

(74) Mandataire: **Tanty, François et al**
**Cabinet Nony**
**3, rue de Penthièvre**
**75008 Paris (FR)**

(56) Documents cités:
**EP-A1- 1 920 760** **FR-A1- 2 863 876**
**FR-A1- 2 908 302**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet une composition cosmétique de revêtement des fibres kératiniques, notamment des cils, se présentant sous forme d'émulsion cire-dans-eau et comprenant au moins un sel mono-sodique de n-stéaroyl-L-acide glutamique et au moins un alkylpolyglycoside étant le cétéaryl glucoside, notamment mis en oeuvre en mélange avec l'alcool cétylstéarylique.

La composition selon l'invention peut être une composition de maquillage ou de soin des fibres kératiniques telles que les cils, les sourcils, les cheveux. Il s'agit plus particulièrement d'une composition de maquillage des fibres kératiniques.

Plus spécialement, la composition selon l'invention est une composition destinée à être appliquée sur les cils, encore appelée « mascara ». Il peut s'agir d'une composition de maquillage, d'une composition cosmétique de revêtement de base appelée également « base coat », d'une composition à appliquer sur une composition cosmétique de revêtement de base, dite encore « top-coat ». Le mascara est plus particulièrement destiné aux cils d'êtres humains, mais également aux faux-cils.

Parmi les formulations de mascaras, on connaît en particulier les mascaras dits « mascaras émulsions » ou « mascaras washable », constitués d'une cire ou mélange de cires dispersées à l'aide d'au moins un tensioactif dans une phase aqueuse, contenant par ailleurs des polymères hydrosolubles et pigments.

Le caractère chargeant des mascaras, figuré par l'épaisseur du dépôt sur le cil, est également une propriété souvent recherchée dans le but de souligner le regard, de le rendre plus intense.

Pour les mascaras de type « washable », le principe pour obtenir un effet volumateur ou chargeant consiste à déposer le maximum de matière sur les cils.

C'est en particulier à travers la quantité de particules solides qui permettent de structurer la composition, que peuvent être ajustées les spécificités d'application recherchées pour les compositions, comme par exemple leur fluidité ou consistance, ainsi que leur pouvoir épaississant, encore appelé pouvoir chargeant ou maquillant.

Ces particules solides, et en particulier les cires, sont généralement dispersées à l'aide d'un système tensioactif. Le choix du système tensioactif est prépondérant dans l'obtention d'une dispersion stable et de consistance élevée, dans la mesure où les tensioactifs jouent un rôle important à l'interface dans les interactions entre particules de cire au sein de la formule.

Ainsi, il est connu par exemple que l'utilisation de tensioactifs non ioniques seuls, non associés avec des tensioactifs ioniques, ne permet pas d'obtenir des dispersions de consistance élevée, et ne conduit donc pas à l'obtention de mascaras suffisamment chargeants. De fait, la texture obtenue avec des tensioactifs non ioniques seuls étant trop fluide, la quantité de matière déposée sur les cils est faible.

Parmi les tensioactifs ou systèmes tensioactifs classiques permettant de conduire à des mascaras présentant un pouvoir chargeant satisfaisant, il est connu en particulier les systèmes tensioactifs à base de stéarate de triéthanolamine, utilisé en couplage ou non avec d'autres tensioactifs.

Cependant, les alcanoamines tertiaires, secondaires et primaires sont de moins en moins recommandables en termes de toxicité.

Par conséquent, il subsiste le besoin de fournir des compositions cosmétiques de revêtement des fibres kératiniques, notamment des cils, exempts de triéthanolamine ou de ses dérivés, et possédant un pouvoir chargeant satisfaisant, permettant notamment de réaliser un maquillage épais des fibres kératiniques, en particulier des cils, dit encore maquillage chargeant.

La présente invention a précisément pour objet de répondre à ce besoin.

Le document WO 02/056940 concerne des compositions moussantes contenant un mélange émulsionnant comprenant un acide aminé acylé, en particulier du cocoacyl glutamate et un oligoglycoside d'alkyle ou d'alkényle.

Le document EP 0 773 017 enseigne des émulsions cosmétiques et pharmaceutiques, notamment des lotions et crèmes, présentant une stabilité améliorée dans le temps et dans des conditions de température élevée, comprenant le mélange d'un alkylpolyglycoside en $C_{16}$-$C_{22}$, d'un alcool gras en $C_{16}$-$C_{22}$ et d'un acylglutamate.

Comme il ressort des exemples présentés ci-après, les inventeurs ont découvert qu'il est possible d'obtenir une émulsion cire-dans-eau présentant de bonnes propriétés chargeantes, en mettant en oeuvre un système émulsionnant comprenant au moins un sel d'acide glutamique particulier et au moins un alkylpolyglycoside particulier.

Plus précisément, les inventeurs ont pu observer que l'association d'au moins un sel mono-sodique de n-stéaroyl-L-acide glutamique et au moins un alkylpolyglycoside étant le cétylstéaryl glucoside, notamment mis en oeuvre en mélange avec l'alcool cétylstéarylique, permet d'obtenir une dispersion stable d'une quantité importante de cires, de la qualité de celles obtenues avec des systèmes émulsionnants à base de stéarate de triéthanolamine.

Au sens de la présente invention, le terme fibres kératiniques couvre les cheveux, les cils et les sourcils, et s'étend également aux postiches et faux-cils synthétiques.

Une « émulsion cire-dans-eau » entend désigner, au sens de la présente invention, une composition comprenant au moins une cire ou mélange de cires dispersée(s) à l'aide d'au moins un tensioactif dans une phase aqueuse

continue.

Par « stable », on entend au sens de la présente invention, une composition qui, après avoir été placée dans une étuve à 45 °C pendant deux mois, ne présente pas, après retour à température ambiante, de grains perceptibles au toucher lorsqu'une couche fine de la composition est cisaillée entre les doigts.

Ainsi, l'invention concerne, selon un de ses aspects, une composition de revêtement des fibres kératiniques, notamment des cils, se présentant sous la forme d'une émulsion cire-dans-eau, et comprenant l'association d'au moins un sel mono-sodique de stéaroyl-L-acide glutamique et d'au moins un alkylpolyglycoside étant le cétylstéaryl glucoside, notamment mis en oeuvre en mélange avec l'alcool cétylstéarylique. la ou lesdites cires étant distincte(s) des alcools gras, notamment des alcools gras comprenant de 10 à 30 atomes de carbone, notamment de 12 à 22 atomes de carbone.

Avantageusement, une composition conforme à l'invention peut comprendre moins de 1 %, de préférence moins de 0,5 % en poids de triéthanolamine ou de ses dérivés, et mieux, être exempte de triéthanolamine ou de ses dérivés.

De manière particulièrement avantageuse, une composition de l'invention peut comprendre une teneur en cires supérieure ou égale à 15 %, de préférence supérieure ou égale à 18 %, et encore plus préférentiellement supérieure ou égale à 20 % en poids, par rapport au poids total de la composition.

Par la teneur élevée de cires qu'elle peut incorporer, une composition selon l'invention présente ainsi une texture suffisamment épaisse pour obtenir un dépôt chargeant, volumateur sur les cils.

Selon un mode de réalisation particulier, les compositions selon l'invention présentent des viscosités, mesurées à température ambiante au Rheomat 180, comprises entre 2 et 25 Pa.s.

Plus précisément, les viscosités sont mesurée à 25 °C à l'aide d'un Rhéomat 180 (Société LAMY) équipé d'un mobile MS-R1, MS-R2, MS-R3, MS-R4 ou MS-R5 choisi en fonction de la consistance de la composition, tournant à une vitesse de rotation de 200 t.min-1. La mesure est prise après 10 min de rotation. Les mesures de viscosité sont réalisées au maximum 1 semaine après la préparation de la composition.

De telles valeurs de viscosités traduisent une consistance élevée des compositions selon l'invention, particulièrement adaptée au maquillage des cils, favorisant en particulier une application facile sur les cils ainsi que l'obtention d'un dépôt lisse et homogène.

Selon un autre de ses aspects, l'invention concerne un procédé cosmétique de maquillage et/ou de soin non thérapeutique des fibres kératiniques, notamment des cils, comprenant l'application sur lesdites fibres kératiniques d'une composition telle que décrite précédemment.

La présente invention vise également l'utilisation d'une composition telle que décrite précédemment, pour obtenir sur les fibres kératiniques, en particulier sur les cils, un maquillage chargeant.

Au sens de la présente invention, on entend qualifier par le terme « chargeant » la notion d'un maquillage épais des cils.

## DERIVE D'ACIDE GLUTAMIQUE

Le système émulsionnant d'une composition selon l'invention comprend au moins un sel mono-sodique de n-stéaroyl-L-acide glutamique

Selon un mode de réalisation particulier, on utilise le sel mono-sodique de n-stéaroyl-L-acide glutamique, plus particulièrement celui commercialisé par la société AJINOMOTO sous la référence Amisoft HS 11.

Le sel mono-sodique d'acide glutamique peut être présent dans une composition de l'invention en une teneur allant de 0,1 à 2 % en poids, de préférence de 0,2 à 1 % en poids par rapport au poids total de la composition.

Selon un mode de réalisation particulier, le sel monosodique d'acide glutamique est présent dans la phase grasse ou alternativement dans la phase aqueuse, et avantageusement dans la phase aqueuse.

## ALKYLPOLYGLYCOSIDE

Le système émulsionnant d'une composition selon l'invention comprend au moins un tensioactif de type alkylpolyglycoside. étant le cétylstéaryl glucoside, notamment mis en oeuvre avec l'alcool cétylstéarylique

Au sens de la présente invention, on entend par « alkylpolyglycoside », un alkylmonooside (degré de polymérisation 1) ou alkylpolyoside (degré de polymérisation supérieur à 1).

Les mélanges émulsionnants alcool gras/alkylpolyglycoside tels que définis ci-dessus sont connus en tant que tels. Ils sont décrits notamment dans les demandes WO 92/06778, WO 95/13863 et WO 98/47610 et préparés selon les procédés de préparation indiqués dans ces documents.

Parmi les mélanges alcool gras/alkylpolyglycoside particulièrement préférés, on peut citer le produit suivant vendu par la société SEPPIC sous l'appellation:

- Alcool cétylstéarylique/Cétylstéarylglucoside - MONTANOV 68®.

Selon un mode de réalisation particulier, l'alkylpolyglycoside mis en oeuvre dans une composition selon l'invention est le cétylstéaryl glucoside. Il est avantageusement utilisé sous la forme d'un mélange avec l'alcool cétylstéarylique, également nommé alcool cétéarylique.

Selon un mode de réalisation particulier de l'invention, on utilise ainsi le mélange alcool cétylstéarylique/cétylstéarylglucoside, commercialisé par la société SEPPIC sous la dénomination MONTANOV 68®, constitué d'environ 20 % de cétylstéaryl glucoside et d'environ 80 % d'alcool cétylstéarylique.

Le mélange alcool gras/alkylpolyglycoside peut être présent dans une composition de l'invention en une teneur allant de 1 à 10 % en poids et plus préférentiellement de 2 à 8 % en poids par rapport au poids total de l'émulsion.

Plus particulièrement, le tensioactif de type alkylpolyglycoside peut être présent dans une composition de l'invention en une teneur allant de 0,2 à 2 %, de préférence de 0,3 à 1,6 % en poids par rapport au poids total de la composition.

Selon un mode de réalisation particulier, une composition conforme à l'invention peut comprendre ledit alkylpolyglycoside et ledit sel mono-sodique d'acide glutamique dans un rapport pondéral alkylpolyglycoside/dérivé d'acide glutamique supérieur ou égal à 0,2, avantageusement supérieur ou égal à 0,3, en particulier inférieur à 2, et plus particulièrement inférieur à 1,6.

Dans le cas particulier où l'alkylpolyglycoside est mis en oeuvre en association avec au moins un alcool gras tel que décrit précédemment, une composition de l'invention peut alors avantageusement comprendre ledit mélange d'alkylpolyglycoside et d'alcool gras et ledit sel mono-sodique d'acide glutamique dans un rapport pondéral (alkylpolyglycoside + alcool gras)/dérivé d'acide glutamique supérieur ou égal 2, avantageusement supérieur ou égal à 2,5, en particulier inférieur à 10, et plus particulièrement inférieur à 8.

Selon un mode de réalisation particulier, le système émulsionnant d'une composition selon l'invention comprend l'association de sel mono-sodique de n-stéaroyl-L-acide glutamique, plus particulièrement celui commercialisé par la société AJINOMOTO sous la référence Amisoft HS 11 ; avec un mélange d'alcool cétylstéarylique/cétylstéaryl glucoside, plus particulièrement celui vendu commercialisé par la société SEPPIC sous la dénomination MONTANOV 68®.

Selon un mode de réalisation particulier, l'association selon l'invention d'un sel mono-sodique de n-stéaroyl-L-acide glutamique et de l'alkylpolyglycoside défini précédemment peut constituer le système tensioactif principal de la composition.

Par « système tensioactif principal », on entend un système qui, en son absence, ne conduit pas à la formation d'une composition stable.

Selon un mode de réalisation particulier, l'association selon l'invention d'un sel mono-sodique de n-stéaroyl-L-acide glutamique et d'un alkylpolyglucoside étant le cétylstéaryl glucoside, notamment mis en oeuvre en mélange avec l'alcool cétylstéarylique peut constituer l'unique système tensioactif de la composition.

Par « unique » on entend que tout éventuel système tensioactif additionnel est présent en une teneur n'excédant pas 1 %, et de préférence n'excédant pas 0,5 %. De préférence encore, par « unique » on désigne une absence totale de tout autre système tensioactif.

Les compositions selon l'invention comprennent bien entendu un milieu physiologiquement acceptable, c'est-à-dire compatible avec une application sur les matières kératiniques, en particulier sur les cils.

## Phase aqueuse

La composition selon l'invention comprend une phase aqueuse, qui forme la phase continue de l'émulsion cire-dans-eau de l'invention.

Par composition à phase continue aqueuse, on entend que la composition présente une conductivité, mesurée à 25 °C, supérieure ou égale à 23 $\mu$S/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

La phase aqueuse comprend de l'eau et/ou au moins un solvant hydrosoluble.

Par « solvant hydrosoluble », on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions conformes à l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en $C_3$-$C_4$ et les aldéhydes en $C_2$-$C_4$.

La phase aqueuse (eau et éventuellement le solvant miscible à l'eau) est généralement présente dans la composition selon la présente demande en une teneur allant de 30 % à 95 % en poids, par rapport au poids total de la

composition, de préférence allant de 40 % à 80 % en poids, et préférentiellement allant de 45 % à 60 % en poids.

**Cires**

Les compositions selon l'invention comprennent d'une manière générale une cire ou un mélange de cires, sous forme d'une dispersion aqueuse de particules de cire.

Comme précisé précédemment, une émulsion cire-dans-eau selon l'invention peut comprendre au moins 15 % en poids en cires, de préférence au moins 18 % en poids, et encore plus préférentiellement au moins 20 % en poids en cires, par rapport au poids total de la composition.

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 120 °C, à l'exception des alcools gras, tels que décrits ci-après, notamment des alcools gras présentant de 10 à 30 atomes de carbone et notamment de 12 à 22 atomes de carbone.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur à 45 °C environ, et en particulier supérieur à 55 °C.

Le point de fusion de la cire peut être mesuré à l'aide d'un calorimètre à balayage différentiel (D.S.C.), par exemple le calorimètre vendu sous la dénomination DSC 30 par la société METLER.

Le protocole de mesure est le suivant :

Un échantillon de 15 mg de produit disposé dans un creuset est soumis à une première montée en température allant de 0 °C à 120 °C, à la vitesse de chauffe de 10 °C/minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, déformables ou non à température ambiante, d'origine animale, végétale, minérale ou de synthèse et leurs mélanges.

La cire peut également présenter une dureté allant de 0,05 MPa à 30 MPa, et de préférence allant de 6 MPa à 15 MPa. La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :

La cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression maximale mesurée divisée par la surface du cylindre du texturomètre en contact avec la cire.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en $C_8$-$C_{32}$.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.

On peut également utiliser les cires obtenues par transesterification et hydrogénation d'huiles végétales, telles que l'huile de ricin ou d'olive, comme les cires vendues sous les dénominations de Phytowax ricin 16L64® et 22L73®

et Phytowax Olive 18L57 par la société SOPHIM. De telles cires sont décrites dans la demande FR-A- 2792190.

On peut aussi utiliser des cires siliconées qui peuvent être avantageusement des polysiloxanes substitués, de préférence à bas point de fusion. Il s'agit notamment de polysiloxanes linéaires substitués constitués essentiellement (les groupes terminaux mis à part) de motifs de formules II et III, dans les proportions molaires respectives m et n :

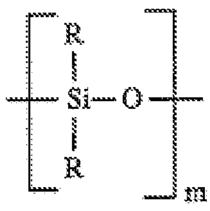

et

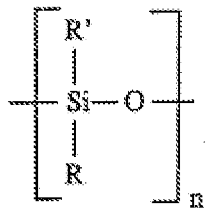

(II) (III)

dans lesquelles :

- chaque substituant R est défini comme précédemment,
- chaque R' représente indépendamment un alkyle (linéaire ou ramifié) éventuellement insaturé, ayant 6-30 atomes de carbone, ou bien un groupement -X-R", chaque X représentant indépendamment :

  -O-,

  $-(CH_2)_a-O-CO-$,

  $-(CH_2)_b-CO-O-$,

  a et b représentent indépendamment des nombres pouvant varier de 0 à 6, et
- chaque R" représente indépendamment un groupement alkyle, éventuellement insaturé, ayant 6 à 30 atomes de carbone,
- m est un nombre pouvant varier de 0 à 400, et en particulier de 0 à 100,
- n est un nombre pouvant varier de 1 à 200, et en particulier de 1 à 100,

la somme (m + n) étant inférieure à 400, et en particulier inférieure ou égale à 100.

Ces cires de silicones sont connues ou peuvent être préparées selon les méthodes connues. Parmi les cires de silicones commerciales de ce type, on peut citer notament celles vendues sous les dénominations Abilwax 9800, 9801 ou 9810 (GOLDSCHMIDT), KF910 et KF7002 (SHIN ETSU), ou 176-1118-3 et 176-11481 (GENERAL ELECTRIC).

Les cires de silicone utilisables peuvent également être choisies parmi les composés de formule (IV) suivante :

$$R_1-Si(CH_3)_2-O-[Si(R)_2-O-]z-Si(CH_3)_2-R_2 \quad (IV)$$

dans laquelle :

R est défini comme précédemment,

$R_1$ représente un groupement alkyle ayant de 1 à 30 atomes de carbone, un groupement alcoxy ayant de 6 à 30 atomes de carbone, ou un groupement de formule :

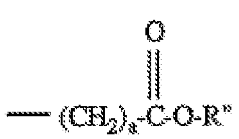

ou

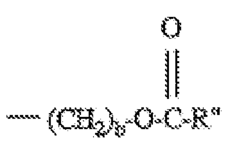

$R_2$ représente un groupement alkyle de 6 à 30 atomes de carbone, un groupement alcoxy ayant de 6 à 30 atomes de carbone ou un groupement de formule :

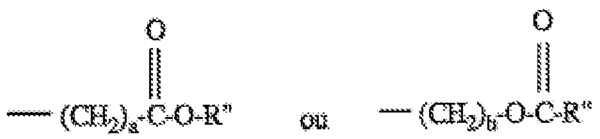

a et b représentant un nombre de 0 à 6,
R" étant un alkyle ayant de 6 à 30 atomes de carbone,
et z est un nombre pouvant varier de 1 à 100.

Parmi les cires de silicone de formule (IV), on citera notamment les alkyl ou alcoxydiméthicones tels que les produits commerciaux suivants : Abilwax 2428, 2434 et 2440 (GOLDSCHMIDT), ou VP 1622 et VP 1621 (WACKER), ainsi que les ($C_{20}$-$C_{60}$) alkyldiméthicones, en particulier les ($C_{30}$-$C_{45}$) alkyldiméthicones comme la cire siliconée vendue sous la dénomination SF-1642 par la société GE-Bayer Silicones.

On peut également utiliser des cires hydrocarbonées modifiées par des groupements siliconés ou fluorés comme par exemple : siliconyl candelilla, siliconyl beeswax et Fluorobeeswax de Koster Keunen.

Les cires peuvent également être choisies parmi les cires fluorées.

Selon un mode de réalisation particulier, les compositions selon l'invention peuvent comprendre au moins une cire dite cire collante c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination « TA-TX2i® » par la société RHEO, équipé d'un mobile en polymère acrylique en forme de cône formant un angle de 45°.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en $C_{20}$-$C_{40}$ (le groupe alkyle comprenant de 20 à 40 atomes de carbone), seul ou en mélange, en particulier un 12-(12'-hydroxystéaryloxy)stéarate d'alkyle en $C_{20}$-$C_{40}$.

Une telle cire est notamment vendue sous les dénominations « Kester Wax K 82 P® » et « Kester Wax K 80 P® » par la société KOSTER KEUNEN.

Les cires citées ci-dessus présentent généralement un point de fusion commençante inférieur à 45 °C.

La ou les cires peu(ven)t être présente(s) sous forme d'une microdispersion aqueuse de cire. On entend par microdispersion aqueuse de cire, une dispersion aqueuse de particules de cire, dans laquelle la taille, exprimée en diamètre « effectif » moyen en volume D[4,3], desdites particules de cire est inférieure ou égale à environ 1 $\mu$m.

Les microdispersions de cire sont des dispersions stables de particules colloïdales de cire, et sont notamment décrites dans « Microemulsions Theory and Practice », L.M. Prince Ed., Academic Press (1977) pages 21-32.

En particulier, ces microdispersions de cire peuvent être obtenues par fusion de la cire en présence d'un

tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 $\mu$m (notamment allant de 0,02 $\mu$m à 0,99 $\mu$m), de préférence inférieures à 0,5 $\mu$m (notamment allant de 0,06 $\mu$m à 0,5 $\mu$m).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

__Composés pâteux__

La composition selon l'invention peut comprendre en outre au moins un composé pâteux.

Par « composé pâteux » au sens de la présente invention, on entend un composé gras lipophile à changement d'état solide/liquide réversible et comportant à la température de 23 °C, une fraction liquide et une fraction solide.

En d'autres termes, la température de fusion commençante du composé pâteux est inférieure à 23 °C. La fraction liquide du composé pâteux, mesurée à 23 °C, représente de 20 à 97 % en poids du composé pâteux. Cette fraction liquide à 23 °C représente plus préférentiellement de 25 à 85 %, et mieux de 30 à 60 % en poids du composé pâteux.

La fraction liquide en poids du composé pâteux à 23 °C est égale au rapport de l'enthalpie de fusion consommée à 23 °C sur l'enthalpie de fusion du composé pâteux.

L'enthalpie de fusion consommée à 23 °C est la quantité d'énergie absorbée par l'échantillon pour passer de l'état solide à l'état qu'il présente à 23 °C constitué d'une fraction liquide et d'une fraction solide.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10 °C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

La fraction liquide du composé pâteux, mesurée à 32 °C, représente de préférence de 40 à 100 % en poids du composé pâteux, mieux encore de 50 à 100 % en poids du composé pâteux. Lorsque la fraction liquide du composé pâteux mesurée à 32 °C est égale à 100 %, la température de la fin de la plage de fusion du composé pâteux est inférieure ou égale à 32 °C.

La fraction liquide du composé pâteux, mesurée à 32 °C, est égale au rapport de l'enthalpie de fusion consommée à 32 °C sur l'enthalpie de fusion du composé pâteux. L'enthalpie de fusion consommée à 32 °C est calculée de la même façon que l'enthalpie de fusion consommée à 23 °C.

Le composé pâteux a de préférence une dureté à 20 °C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20 °C au centre de 5 échantillons. Le cylindre est introduit dans chaque échantillon, la profondeur de pénétration étant de 0,3 mm. La valeur relevée de la dureté est celle du pic maximum.

Le composé pâteux peut être choisi parmi les composés synthétiques et les composés d'origine végétale. Un composé pâteux peut être obtenu par synthèse à partir de produits de départ d'origine végétale.

Le composé pâteux est avantageusement choisi parmi :

- la lanoline et ses dérivés tels que l'alcool de lanoline, les lanolines oxyéthylénées, la lanoline acétylée, les esters de lanoline tels que le lanolate d'isopropyle, les lanolines oxypropylénées,
- les composés siliconés polymères ou non-polymères comme les polydiméthysiloxanes de masses moléculaires élevées, les polydiméthysiloxanes à chaînes latérales du type alkyle ou alcoxy ayant de 8 à 24 atomes de carbone, notamment les stéaryl diméthicones,
- les composés fluorés polymères ou non-polymères,
- les polymères vinyliques, notamment

- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyle ayant de préférence un groupement alkyle en $C_8$-$C_{30}$,
- les oligomères homo et copolymères d'esters vinyliques ayant des groupements alkyles en $C_8$-$C_{30}$,
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,

- les esters et les polyesters,
- et leurs mélanges.

Le composé pâteux peut être un polymère, notamment hydrocarboné.
Un composé pâteux siliconé et fluoré préféré est le polyméthyl-trifluoropropyl-méthylalkyl-diméthylsiloxane, fabriqué sous la dénomination X22-1088 par SHIN ETSU.
Lorsque le composé pâteux est un polymère siliconé et/ou fluoré, la composition comprend avantageusement un agent compatibilisant tel que les esters à courte chaîne comme le néopentanoate d'isodécyle.
Parmi les polyéthers liposolubles, on peut notamment citer les copolymères d'oxyde d'éthylène et/ou d'oxyde de propylène avec des oxydes d'alkylène en $C_6$-$C_{30}$. De préférence, le rapport pondéral de l'oxyde d'éthylène et/ou de l'oxyde de propylène avec les oxydes d'alkylène dans le copolymère est de 5:95 à 70:30. Dans cette famille, on citera notamment les copolymères blocs comprenant des blocs d'oxydes d'alkylène en $C_6$-$C_{30}$ ayant un poids moléculaire allant de 1 000 à 10 000, par exemple un copolymère bloc polyoxyéthylène/polydodécylène glycol tel que les éthers de dodécanediol (22 mol) et de polyéthylène glycol (45 motifs oxyéthylène ou OE) commercialisés sous la marque ELFACOS ST9 par Akzo Nobel.
Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras comme l'acide stéarique, l'acide caprique, l'acide stéarique, l'acide isostéarique et l'acide 12-hydroxystéarique, tels que ceux notamment commercialisés sous la marque Softisan 649 par la société Sasol ;
- les esters de phytostérol ;
- les esters de pentaérythritol ;
- les esters formés à partir :

    - d'au moins un alcool en $C_{16-40}$, l'un au moins des alcools étant un alcool de Guerbet et
    - d'un dimère diacide formé à partir d'au moins un acide gras insaturé en $C_{18-40}$,

  comme l'ester de dimère d'acides gras de tallol comprenant 36 atomes de carbone et d'un mélange i) d'alcools de Guerbet comprenant 32 atomes de carbone et ii) d'alcool béhénylique ; l'ester de dimère d'acide linoléique et d'un mélange de deux alcools de Guerbet, le 2-tétradécyl-octadécanol (32 atomes de carbone) et le 2-hexadécyl-eicosanol (36 atomes de carbone) ;
- les polyesters non réticulés résultant de la polycondensation entre un acide dicarboxylique ou un acide polycarboxylique, linéaire ou ramifié, en $C_4$-$C_{50}$, et un diol ou un polyol en $C_2$-$C_{50}$ ;
- les polyesters qui résultent de l'estérification entre un acide polycarboxylique et un ester d'acide carboxylique hydroxylé aliphatique comme le Risocast DA-L et le Risocast DA-H commercialisés par la société japonaise KOKYU ALCOHOL KOGYO, qui sont des esters résultant de la réaction d'estérification de l'huile de ricin hydrogénée avec l'acide dilinoléïque ou l'acide isostéarique ; et
- les esters aliphatiques d'ester résultant de l'estérification entre un ester d'acide carboxylique hydroxylé aliphatique et un acide carboxylique aliphatique, par exemple celui vendu sous la dénomination commerciale Salacos HCIS (V)-L par la société Nishing Oil.

Un alcool de Guerbet est le produit réactionnel de la réaction de Guerbet bien connue de l'homme du métier. Il s'agit d'une réaction transformant un alcool aliphatique primaire en son alcool dimère $\beta$-alkylé avec perte d'un équivalent d'eau.
Les acides carboxyliques aliphatiques décrits ci-dessus comprennent généralement de 4 à 30 et de préférence de 8 à 30 atomes de carbone. Ils sont choisis de préférence parmi l'acide héxanoïque, l'acide heptanoïque, l'acide octanoïque, l'acide 2-éthylhexanoïque, l'acide nonanoïque, l'acide décanoïque, l'acide undécanoïque, l'acide dodécanoïque, l'acide tridécanoïque, l'acide tétradécanoïque, l'acide pentadécanoïque, l'acide hexadécanoïque, l'acide hexyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

Les acides carboxyliques aliphatiques sont de préférence ramifiés.

Les esters d'acide carboxylique aliphatique hydroxylé sont avantageusement issus d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. Les esters d'acide carboxylique aliphatique hydroxylé sont notamment choisis parmi :

a) les esters, partiels ou totaux, d'acides monocarboxyliques aliphatiques monohydroxylés linéaires, saturés ;
b) les esters, partiels ou totaux, d'acides monocarboxyliques aliphatiques monohydroxylés insaturés ;
c) les esters, partiels ou totaux, de polyacides carboxyliques aliphatiques monohydroxylés saturés ;
d) les esters, partiels ou totaux, de polyacides carboxyliques aliphatiques polyhydroxylés saturés ;
e) les esters, partiels ou totaux, de polyols aliphatiques en $C_2$ à $C_{16}$ ayant réagi avec un acide mono- ou un polycarboxylique aliphatique mono- ou polyhydroxylé,
f) et leurs mélanges.

Les esters aliphatiques d'ester sont avantageusement choisis parmi :

- l'ester résultant de la réaction d'estérification de l'huile de ricin hydrogénée avec l'acide isostéarique dans les proportions 1 pour 1 (1/1), qui est appelé monoisostéarate d'huile de ricin hydrogénée,
- l'ester résultant de la réaction d'estérification de l'huile de ricin hydrogénée avec l'acide isostéarique dans les proportions 1 pour 2 (1/2), qui est appelé le diisostéarate d'huile de ricin hydrogénée,
- l'ester résultant de la réaction d'estérification de l'huile de ricin hydrogénée avec l'acide isostéarique dans les proportions 1 pour 3 (1/3), qui est appelé le triisostéarate d'huile de ricin hydrogénée,
- et leurs mélanges.

De préférence, le composé pâteux est choisi parmi les composés d'origine végétale.

Parmi ceux-ci, on peut notamment citer l'huile de jojoba isomérisée telle que l'huile de jojoba partiellement hydrogénée isomérisée trans fabriquée ou commercialisée par la société Desert Whale sous la référence commerciale Iso-Jojoba-50®, la cire d'orange comme, par exemple, celle qui est commercalisée sous la référence Orange Peel Wax par la société Koster Keunen, le beurre de cupuacu (Rain forest RF3410), le beurre de murumuru (murumuru butter de la société Beraca Sabara), le beurre de karité, l'huile d'olive partiellement hydrogénée comme, par exemple, le composé commercialisé sous la référence Beurrolive par la société Soliance, le beurre de cacao, l'huile de mangue comme, par exemple, la Lipex 302 de la société Aarhuskarlshamn.

Selon un mode de réalisation particulier, une composition selon l'invention comprend du beurre de karité.

Le ou les composés pâteux sont présents de préférence en une quantité supérieure allant de 0,5 % à 10 % en poids, notamment de 0,5 % à 6 % en poids, par rapport au poids total de la composition.

## Tensioactifs additionnels et co-tensioactifs

La composition selon l'invention peut comprendre outre l'association des tensioactifs selon l'invention, au moins un agent tensioactif additionnel et/ou au moins un co-tensioactif, en particulier choisi de manière appropriée pour l'obtention d'une émulsion cire-dans-eau.

Les agents tensioactifs additionnels peuvent être choisis parmi des agents tensioactifs anioniques, non ioniques, cationiques, amphotères ou zwitterioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs additionnels peuvent être choisis parmi :

a) les agents tensioactifs non ioniques de HLB supérieur ou égal à 8 à 25 °C, utilisés seuls ou en mélange ; on peut citer notamment :

- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool stéarylique à 20 motifs oxyéthylène (nom CTFA « Steareth-20 ») comme le BRIJ 78 commercialisé par la société UNIQEMA, l'éther oxyéthyléné de l'alcool cétéarylique à 30 groupes oxyéthylénés (nom CTFA « Ceteareth-30 ») et l'éther oxyéthyléné du mélange d'alcools gras en $C_{12}$-$C_{15}$ comportant 7 groupes oxyéthylénés (nom CTFA « $C_{12-15}$ Pareth-7 » commercialisé sous la dénomination « NEODOL 25-7 »® par SHELL CHEMICALS) ;

- les esters d'acide gras (notamment d'acide en $C_8$-$C_{24}$, et de préférence en $C_{16}$-$C_{22}$) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P par la société ICI UNIQUEMA ;
- les esters d'acide gras (notamment d'acide en $C_8$-$C_{24}$, et de préférence en $C_{16}$-$C_{22}$) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination « Simulsol 220 TM » par la société SEPPIC ; le stéarate de glycéryle polyéthoxylé à 30 groupes d'oxyde d'éthylène comme le produit TAGAT S vendu par la société GOLDSCHMIDT, l'oléate de glycéryle polyéthoxylé à 30 groupes d'oxyde d'éthylène comme le produit TAGAT O vendu par la société GOLDSCHMIDT, le cocoate de glycéryle polyéthoxylé à 30 groupes d'oxyde d'éthylène comme le produit VARIONIC LI 13 vendu par la société SHEREX, l'isostéarate de glycéryle polyéthoxylé à 30 groupes d'oxyde d'éthylène comme le produit TAGAT L vendu par la société GOLDSCHMIDT et le laurate de glycéryle polyéthoxylé à 30 groupes d'oxyde d'éthylène comme le produit TAGAT I de la société GOLDSCHMIDT ;
- les esters d'acide gras (notamment d'acide en $C_8$-$C_{24}$, et de préférence en $C_{16}$-$C_{22}$) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination « Tween 60 » par la société UNIQUEMA ;
- la diméthicone copolyol, telle que celle vendue sous la dénomination « Q2-5220 » par la société DOW CORNING ;
- la diméthicone copolyol benzoate (FINSOLV SLB 101 et 201 de la société FINTEX) ;
- les copolymères d'oxyde propylène et d'oxyde d'éthylène, également appelés polycondensats OE/OP, comme par exemple les polycondensats tribloc polyéthylène glycol/polypropylène glycol/polyéthylène glycol vendus sous les dénominations « SYNPERONIC » comme les « SYNPERONIC PE/ L44 » et « SYNPERONIC PE/F127 » par la société ICI ;

et leurs mélanges.

b) les agents tensioactif non ioniques de HLB inférieur à 8 à 25 °C, éventuellement associés à un ou plusieurs agents tensioactif non ioniques de HLB supérieur à 8 à 25 °C, tels que :

- les esters et éthers d'oses tels que les stéarates de sucrose, cocoate de sucrose, stéarate de sorbitan et leurs mélanges comme l'Arlatone 2121 commercialisé par la société ICI ;
- les esters d'acides gras (notamment d'acide en $C_8$-$C_{24}$, et de préférence en $C_{16}$-$C_{22}$) et de polyol, notamment de glycérol ou de sorbitol, tels que stéarate de glycéryle, tel que le produit vendu sous la dénomination TEGIN M par la société GOLDSCHMIDT, laurate de glycéryle tel que le produit vendu sous la dénomination IMWITOR 312 par la société HULS, stéarate de polyglycéryl-2, tristéarate de sorbitan, ricinoléate de glycéryle ;
- les éthers oxyéthylénés et/ou oxypropylénés tels que l'éther oxyéthyléné de l'alcool stéarylique à 2 motifs oxyéthylène (nom CTFA « Steareth-2 ») tel que le BRIJ 72 commercialisé par la société UNIQEMA ;
- le mélange de cyclométhicone/diméthicone copolyol vendu sous la dénomination « Q2-3225C » par la société DOW CORNING.

c) les tensioactifs anioniques tels que :

- les sels d'acides gras en $C_{16}$-$C_{30}$ notamment ceux dérivant des amines, comme le stéarate de triéthanolamine et/ou le stéarate d'amino-2-méthyl-2-propane diol-1,3 ; mais de préférence la composition selon la présente demande comprend moins 1% de stéarate de triéthanolamine ;
- les sels d'acides gras polyoxyéthylénés notamment ceux dérivant des amines ou les sels alcalins, et leurs mélanges ;
- les esters phosphoriques et leurs sels tels que le « DEA oleth-10 phosphate » (Crodafos N 10N de la société CRODA), le cetyl phosphate (Amphisol K de la société DSM Nutritionnal Products) ;
- les sulfosuccinates tels que le « Disodium PEG-5 citrate lauryl sulfosuccinate » et le « Disodium ricinoleamido MEA sulfosuccinate » ;
- les alkyléthersulfates tels que le lauryl éther sulfate de sodium ;
- les iséthionates ;

- les acylglutamates tels que le « Disodium hydrogenated tallow glutamate » (AMISOFT HS-21 R commercialisé par la société AJINOMOTO) et leurs mélanges.

La composition conforme à l'invention peut également contenir, outre le dérivé d'acide glutamique, un ou plusieurs tensioactifs amphotères comme les N-acyl-aminoacides tels que les N-alkyl-aminoacétates et le cocoamphodiacétate disodique et les oxydes d'amines tels que l'oxyde de stéaramine ou encore des tensioactifs siliconés comme les diméthicone copolyols phosphates tels que celui vendu sous la dénomination PECOSIL PS 100® par la société PHOENIX CHEMICAL.

Selon un mode de réalisation particulier, le tensioactif additionnel peut être le mono et/ou distéarate de glycéryle.

Selon un mode de réalisation préféré, une composition conforme à l'invention comprend moins de 1 %, de préférence moins de 0,5 % en poids de triéthanolamine ou de ses dérivés, et mieux, est exempte de triéthanolamine ou de ses dérivés.

Les co-tensioactifs peuvent notamment être choisis parmi les alcools gras, comprenant de préférence de 10 à 30 atomes de carbone. Par alcool gras comprenant de 10 à 30 atomes de carbone, on entend tout alcool gras pur, saturé ou non, ramifié ou non, comportant de 10 à 30 atomes de carbone.

A titre d'exemples d'alcools gras pouvant être utilisés en association avec le(s) alkylpolyglycoside(s) du système émulsionnant selon l'invention, on peut citer les alcools gras linéaires ou ramifiés, d'origine synthétique, ou encore naturelle comme par exemple les alcools provenant de matières végétales (coprah, palmiste, palme, etc) ou animales (suif, etc). Bien entendu, d'autres alcools à longue chaîne peuvent également être utilisés, comme par exemple les étheralcools ou bien encore les alcools dits de Guerbet. Enfin, on peut également utiliser certaines coupes plus ou moins longues d'alcools d'origine naturelle, comme par exemple coco ($C_{12}$ à $C_{16}$) ou suif ($C_{16}$ à $C_{18}$) ou des composés type diols ou cholesterol.

On utilise de préférence un alcool gras comprenant de 10 à 26 atomes de carbone, de préférence de 10 à 24 atomes de carbone, et plus préférentiellement de 12 à 22 atomes de carbone.

A titre d'exemples particuliers d'alcools gras utilisables dans le cadre de la présente invention, on peut notamment citer l'alcool laurique, myristique, cétylique, stéarylique, isostéarylique, palmitique, oléique, cétéarylique (mélange d'alcool cétylique et stéarylique), béhénique, érucique, arachidylique et leurs mélanges. On utilise de préférence l'alcool cétéarylique.

De tels alcools gras sont notamment commercialisés sous la dénomination NAFOL par la société SASOL.

Comme décrit précédemment, ces alcools gras peuvent être mis en oeuvre conjointement avec le(s) alkylpolyglycoside(s) selon l'invention, dans des mélanges commercialement disponibles alcool gras/alkylpolyglycoside, tels que par exemple le mélange d'alcool cétylstéarylique et de cétylstéarylglucoside vendu par la société SEPPIC, sous la référence MONTANOV 68®.

Le ou les co-tensioactifs peuvent être présents en une teneur allant de 0,1 à 10 % en poids, de préférence de 0,5 à 8 % en poids, et plus préférentiellement de 1 à 6 % en poids par rapport au poids total de la composition.

La teneur globale en agents tensioactifs dans une composition de l'invention peut aller de 0,5 à 15 % en poids par rapport au poids total de la composition, de préférence de 1 à 10 % en poids.

## Polymère filmogène

Une composition selon la présente invention peut en outre comprendre au moins un polymère filmogène.

Parmi les polymères filmogènes utilisables dans les compositions de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle,

en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, $K^+$, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de l'invention, le polymère filmogène peut être un polymère hydrosoluble et peut être alors présent dans la phase continue aqueuse d'une émulsion selon l'invention.

Selon une autre variante, le polymère filmogène peut être un polymère solubilisé dans une phase grasse liquide comprenant des huiles ou solvants organiques tels que ceux décrits par la suite (on dit alors que le polymère filmogène est un polymère liposoluble). De préférence, la phase grasse liquide comprend une huile volatile, éventuellement en mélange avec une huile non volatile, les huiles pouvant être choisies parmi les huiles citées ci-dessous.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2 000 à 500 000 et de préférence de 4 000 à 200000.

On peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 2 à 24 atomes de carbone.

Comme exemples d'homopolymères liposolubles, on peut citer notamment: les polylaurate de vinyle et le poly(méth)acrylates de lauryle, ces poly(méth)acrylates pouvant être réticulés à l'aide de diméthacrylate de l'éthylène glycol ou de tétraéthylène glycol.

Selon un mode de réalisation avantageux, une composition selon l'invention comprend au moins un polymère filmogène polylaurate de vinyle.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de « MDTQ », la résine étant décrite en fonction des différentes unités monomèriques siloxane qu'elle comprend, chacune des lettres « MDTQ » caractérisant un type d'unité.

A titre d'exemples de résines polyméthylsilsesquioxanes commercialement disponibles, on peut citer celles qui sont commercialisés par la société Wacker sous la référence Resin MK tels que la Belsil PMS MK, et par la société SHIN-ETSU sous les références KR-220L.

Comme résines siloxysilicates, on peut citer les résines triméthylsiloxysilicate (TMS) telles que celles commercialisées sous la référence SR1000 par la société General Electric ou sous la référence TMS 803 par la société Wacker.

On peut encore citer les résines triméthylsiloxysilicate commercialisées dans un solvant tel que la cyclomethicone, vendues sous la dénomination «KF-7312J» par la société Shin-Etsu, « DC 749 », « DC 593 » par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5,162,410 ou encore les copolymères siliconés issus de la réaction d'un résine de silicone, telle que celles décrite plus haut, et d'un diorganosiloxane tels que décrits dans le document WO 2004/073626.

On peut également utiliser les polyamides siliconés du type polyorganosiloxane tels que ceux décrits dans les documents US-A-5,874,069, US-A-5,919,441, US-A-6,051,216 et US-A-5,981,680.

Ces polymères siliconés peuvent appartenir aux deux familles suivantes :

- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1 411 069 ou WO 04/028488.

Le polymère filmogène peut être également présent dans une composition de l'invention sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO ; Syntran 5760® par la société Interpolymer, Allianz OPT par la société ROHM & HAAS, les dispersions aqueuses de polymères acryliques ou styrène/acrylique vendues sous le nom de marque JONCRYL® par la société JOHNSON POLYMER ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Sancure 861®, Sancure 878® et Sancure 2060® par la société GOODRICH, Impranil 85® par la société BAYER, Aquamere H-1511® par la société HYDROMER ; les sulfopolyesters vendus sous le nom de marque Eastman AQ® par la société Eastman Chemical Products, les dispersions vinyliques comme le Mexomère PAM® de la société CHIMEX et leurs mélanges.

Comme exemples de dispersions non aqueuses de polymère filmogène, on peut citer les dispersions acryliques dans l'isododécane comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

Une composition selon l'invention peut également comprendre en outre un agent plastifiant favorisant la formation d'un film avec le polymère filmogène. Un tel agent plastifiant peut être choisi parmi tous les composés connus de l'homme du métier comme étant susceptibles de remplir la fonction recherchée.

**Gélifiants**

Une composition de l'invention peut également comprendre au moins un gélifiant hydrophile ou hydrosoluble.

Comme gélifiants hydrophiles ou hydrosolubles, on peut citer :

- les homo- ou copolymères d'acides acrylique ou méthacrylique ou leurs sels et leurs esters et en particulier les produits vendus sous les dénominations « VERSICOL F» ou « VERSICOL K» par la société ALLIED COLLOID, « UTRAHOLD 8 » par la société CIBA-GEIGY, les acides polyacryliques de type SYNTHALEN K,
- les copolymères d'acide acrylique et d'acrylamide vendus sous la forme de leur sel de sodium sous les dénominations « RETEN » par la société HERCULES, le polyméthacrylate de sodium vendu sous la dénomination « DARVAN

N°7 » par la société VANDERBILT, les sels de sodium d'acides polyhydroxycarboxyliques vendus sous la dénomination « HYDAGEN F » par la société HENKEL,

- les copolymères acides polyacryliques/acrylates d'alkyle de type PEMULEN,
- l'AMPS (Acide polyacrylamidométhyl propane sulfonique neutralisé partiellement à l'ammoniaque et hautement réticulé) commercialisé par la société CLARIANT,
- les copolymères AMPS/acrylamide de type SEPIGEL ou SIMULGEL commercialisés par la société SEPPIC, et
- les copolymères AMPS/méthacrylates d'alkyle polyoxyéthylénés (réticulés ou non) et leurs mélanges.

Comme autres exemples de polymères gélifiants hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polyuréthanes associatifs tels que le polymère C16-OE120-C16 de la société SERVO DELDEN (commercialisé sous le nom SER AD FX1100, molécule à fonction uréthane et poids moléculaire moyen en poids de 1300), OE étant un motif oxyéthyléné, le Rhéolate 205 à fonction urée vendu par la société RHEOX ou encore le Rhéolate 208 ou 204 (ces polymères étant vendus sous forme pure) ou le DW 1206B de chez RHOM & HAAS à chaîne alkyle en $C_{20}$ et à liaison uréthane, vendu à 20 % en matière sèche dans l'eau. On peut aussi utiliser des solutions ou dispersions de ces polyuréthanes associatifs notamment dans l'eau ou en milieu hydroalcoolique. A titre d'exemples de tels polymères, on peut citer le SER AD fx1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS,
- les polymères d'origine naturelle, éventuellement modifiés, tels que :
    - les gommes arabiques, la gomme de guar, les dérivés du xanthane, la gomme de karaya ;
    - les alginates et les carraghénanes ;
    - les glycoaminoglycanes, l'acide hyaluronique et ses dérivés ;
    - la résine shellac, la gomme de sandaraque, les dammars, les élémis, les copals ;
    - l'acide désoxyribonucléïque ;
    - les muccopolysaccharides tels que l'acide hyaluronique, les chondroïtines sulfate, et leurs mélanges.

Certains des polymères filmogènes hydrosolubles cités plus haut peuvent également jouer le rôle de gélifiant hydrosoluble.

Les gélifiants hydrophiles peuvent être présents dans les compositions selon l'invention en une teneur allant de 0,05 à 40 % en poids par rapport au poids total de la composition, de préférence de 0,1 à 20 % et mieux de 0,5 à 15 % en poids.

**Huiles et solvants organiques**

Les compositions selon la présente demande peuvent aussi contenir au moins une ou plusieurs huiles ou solvants organiques.

Par « huile ou solvant organique », au sens de la demande, on entend un corps non aqueux liquide à température ambiante (25 °C) et pression atmosphérique (760 mm de Hg).

L'huile peut être choisie parmi les huiles volatiles et/ou les huiles non volatiles, et leurs mélanges.

La ou les huiles peuvent être présentes en une teneur allant de 0,05 % à 15 % en poids, de préférence de 0,1 % à 10 % en poids par rapport au poids total de la composition.

Par « huile volatile », on entend au sens de l'invention une huile susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

En particulier, les huiles volatiles sont choisies parmi les huiles possédant une vitesse d'évaporation supérieure ou égale à 0,002 mg/cm$^2$/min. La vitesse d'évaporation étant mesurée comme suit :

On introduit dans un cristallisoir (diamètre : 7 cm) placé sur une balance se trouvant dans une enceinte d'environ 0,3 m$^3$ régulée en température (25 °C) et en hygrométrie (humidité relative 50 %) 15 g d'huile ou du mélange d'huiles à tester.

On laisse le liquide s'évaporer librement, sans l'agiter, en assurant une ventilation par un ventilateur (vitesse de rotation 2700 tours/minute et de dimensions 80X80X42 mm, par exemple la référence 8550 N de PAPST-MOTOREN, le débit correspond à environ 50 m$^3$/heure) disposé en position verticale au-dessus du cristallisoir contenant le solvant, les pales étant dirigées vers le cristallisoir et à une distance de 20 cm par rapport au fond du cristallisoir.

On mesure à intervalles réguliers la masse d'huile restant dans le cristallisoir. Les vitesses d'évaporation sont exprimées en mg d'huile évaporée par unité de surface (cm$^2$) et par unité de temps (minute).

Par « huile non volatile », on entend une huile restant sur les matières kératiniques à température ambiante et pression atmosphérique au moins plusieurs heures et ayant notamment une pression de vapeur inférieure à $10^{-3}$ mm de Hg (0,13 Pa).

Ces huiles peuvent être des huiles hydrocarbonées, des huiles siliconées, des huiles fluorées, ou leurs mélanges.

On entend par « huile hydrocarbonée », une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars ou de Permetyls, les esters ramifiés en $C_8$-$C_{16}$, le néopentanoate d'iso-hexyle, et leurs mélanges. D'autres huiles hydrocarbonées volatiles comme les distillats de pétrole, notamment ceux vendus sous la dénomination Shell Soit par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 8 centistokes ($8.10^{-6}$ m$^2$/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants volatils fluorés tels que le nonafluorométhoxybutane ou le perfluorométhylcyclopentane.

La composition peut également comprendre au moins une huile ou solvant organique non volatils, et notamment choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triesters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges ;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit ≥ 10, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et

les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- les carbonates,
- les acétales,
- les citrates,
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.
Les huiles fluorées utilisables dans l'invention sont notamment des huiles fluorosiliconées, des polyéthers fluorés, des silicones fluorées telles que décrit dans le document EP-A-847752.

**Matières colorantes**

La composition selon l'invention peut également comprendre au moins une matière colorante, choisie par exemple parmi les pigments, les nacres, les colorants, les matériaux à effet et leurs mélanges.
Ces matières colorantes peuvent être présentes en une teneur allant de 0,01 à 30 % en poids par rapport au poids total de la composition.
Les pigments utiles dans la présente invention peuvent se présenter sous forme de poudre ou de pâte pigmentaire.
Par « colorants », il faut comprendre des composés, généralement organiques, solubles dans au moins une huile ou dans une phase hydroalcoolique.
Par « pigments », il faut comprendre des particules blanches ou colorées, minérales ou organiques, insolubles dans un milieu aqueux, destinées à colorer et/ou opacifier le film résultant.
Par « nacres » ou pigments nacrés, il faut comprendre des particules colorées de toute forme, irisées ou non, notamment produites par certains mollusques dans leur coquille ou bien synthétisées et qui présentent un effet de couleur par interférence optique.
Le pigment peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.
Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.
Ces pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.
Le pigment peut aussi être une laque. Par laque, on entend les colorants insolubilisés adsorbés sur des particules insolubles, l'ensemble ainsi obtenu restant insoluble lors de l'utilisation.
Les substrats inorganiques sur lesquels sont adsorbés les colorants sont par exemple l'alumine, la silice, le borosilicate de calcium et de sodium ou le borosilicate de calcium et d'aluminium, et l'aluminium.
Parmi les colorants organiques, on peut citer le carmin de cochenille. On peut également citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200),

D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de zirconium ou de cérium, ainsi que les oxydes de fer ou de chrome, le violet de manganèse, le bleu outremer, l'hydrate de chrome et le bleu ferrique, le dioxyde de titane. Les pigments minéraux suivants peuvent aussi être utilisés : $Ta_2O_5$, $Ti_3O_5$, $Ti_2O_3$, TiO, $ZrO_2$ en mélange avec $TiO_2$, $ZrO_2$, $Nb_2O_5$, $CeO_2$, ZnS.

Le pigment peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation, etc). Ils s'opposent par-là même aux pigments blancs ou colorés qui procurent une teinte uniforme opaque, semi-transparente ou transparente classique.

Il existe deux types de pigments à effets spéciaux, ceux à faible indice de réfraction tels que les pigments fluorescents, photochromes ou thermochromes, et ceux à plus fort indice de réfraction tels que les nacres ou les paillettes.

A titre de pigments à effets spéciaux, on peut citer les pigments nacrés tels que les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriqués selon les procédés décrits par exemple dans le US 6,225,198 ou US 5,990,479, dans les publications qui y sont citées, ainsi que dans les publications suivantes : Dabboussi B.O. et al « (CdSe)ZnS core-shell quantum dots : synthesis and characterisation of a size series of highly luminescent nanocristallites » Journal of phisical chemistry B, vol 101, 1997, pp 9463-9475. et Peng, Xiaogang et al, « Epitaxial Growth of highly Luminescent CdSe/CdS core/shell nanocrystals with photostability and electronic accessibility » Journal of the American Chemical Society, vol 119, N°30, pp 7019-7029.

Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques.

Le pigment peut aussi être un pigment nacré tels que des pigments nacrés blancs par exemple le mica recouvert de titane, ou d'oxychlorure de bismuth, des pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre d'exemple, on peut citer les pigments Cellini commercialisée par Engelhard (Mica-$TiO_2$-laque), Prestige commercialisée par Eckart (Mica-$TiO_2$), Colorona commercialisée par Merck (Mica-$TiO_2$-$Fe_2O_3$).

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

La taille du pigment utile dans le cadre de la présente invention est généralement comprise entre 10 nm et 200 $\mu$m, de préférence entre 20 nm et 80 $\mu$m, et plus préférentiellement entre 30 nm et 50 $\mu$m.

Les pigments peuvent être dispersés dans le produit grâce à un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100

par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy12-stéarique sont de préférence destinés à un milieu hydrocarboné ou fluoré, alors que les mélanges de diméthylsiloxane oxyéthylène/oxypropyléné sont de préférence destinés à un milieu siliconé.

## Pigments enrobés

Selon un mode de réalisation particulièrement avantageux, la composition comprend au moins un pigment enrobé par au moins un composé lipophile, ledit pigment étant notamment choisi parmi les pigments organiques, les pigments composites, les laques et les pigments minéraux, tels que décrits précédemment.

L'enrobage peut aussi comprendre au moins un composé additionnel non lipophile.

Au sens de l'invention, l'« enrobage » d'un pigment selon l'invention désigne de manière générale le traitement en surface total ou partiel du pigment par un agent de surface, absorbé, adsorbé ou greffé sur ledit pigment.

Les pigments traités en surface peuvent être préparés selon des techniques de traitement de surface de nature chimique, électronique, mécano-chimique ou mécanique bien connues de l'homme de l'art. On peut également utiliser des produits commerciaux.

L'agent de surface peut être absorbé, adsorbé ou greffé sur les pigments par évaporation de solvant, réaction chimique et création d'une liaison covalente.

Selon une variante, le traitement de surface consiste en un enrobage particules solides.

L'enrobage peut représenter de 0,1 à 20 % en poids, et en particulier de 0,5 à 5 % en poids du poids total du pigment enrobé.

L'enrobage peut être réalisé par exemple par adsorption d'un agent de surface liquide à la surface des particules solides par simple mélange sous agitation des particules et dudit agent de surface, éventuellement à chaud, préalablement à l'incorporation des particules dans les autres ingrédients de la composition de maquillage ou de soin.

L'enrobage peut être réalisé par exemple par réaction chimique d'un agent de surface avec la surface des particules solides de pigment et création d'une liaison covalente entre l'agent de surface et les particules. Cette méthode est notamment décrite dans le brevet US 4,578,266.

Le traitement de surface chimique peut consister à diluer l'agent de surface dans un solvant volatile, à disperser les pigments dans ce mélange, puis à évaporer lentement le solvant volatile, de manière à ce que l'agent de surface se dépose à la surface des pigments.

## Agent de traitement lipophile ou hydrophobe

Lorsque le pigment comprend un enrobage lipophile, ce dernier est présent dans la phase grasse de la composition selon l'invention.

Selon un mode de réalisation particulier de l'invention, les pigments peuvent être enrobés selon l'invention par au moins un composé choisi parmi les agents de surface siliconés ; les agents de surface fluorés ; les agents de surface fluoro-siliconés ; les savons métalliques, les acides aminés N-acylés ou leurs sels ; la lécithine et ses dérivés ; le trisostéaryle titanate d'isopropyle ; le sébaçate d'isostéaryle ; les cires naturelles végétales ou animales, les cires synthétiques polaires; les esters gras ; les phospholipides, et leurs mélanges.

### *Agent de surface siliconé*

Selon un mode de réalisation particulier, les pigments peuvent être traités en surface totalement ou partiellement avec un composé de nature siliconée.

Les agents de surface siliconés peuvent être choisis parmi les organopolysiloxanes, les dérivés de silanes, les copolymères silicone-acrylate, les résines de silicone, et leurs mélanges.

Par composé organopolysiloxane, on entend un composé ayant une structure comprenant une alternance d'atomes de silicone et d'atomes d'oxygène et comprenant des radicaux organiques liés aux atomes de silicium.

*i) Organopolysiloxane non élastomère*

On peut notamment citer comme organopolysiloxanes non élastomères, les polydiméthylsiloxanes, les polyméthylhydrogénosiloxanes et les polyalcoxydiméthylsiloxanes.

Le groupe alcoxy peut être représenté par le radical R-O- tel que R représente méthyl, éthyl, propyl, butyl ou octyl, des radicaux 2-phényléthyl, 2-phénylpropyl ou 3,3,3-trifluoropropyl, des radicaux aryles tels que phényl, tolyl, xylyl, ou des radicaux aryles substitués tels que phényléthyl.

Une méthode permettant de traiter en surface des pigments par un polyméthylhydrogénosiloxane consiste à disperser les pigments dans un solvant organique, puis à ajouter le composé siliconé. En chauffant le mélange, des liaisons covalentes se créent entre le composé siliconé et la surface du pigment.

Selon un mode préféré de réalisation, l'agent de surface siliconé peut être un organopolysiloxane non élastomères, notamment choisi parmi les polydiméthylsiloxanes.

*ii) Alkylsilanes et alcoxysilanes*

Des silanes à fonctionnalité alcoxy sont notamment décrits par Witucki dans « A silane primer, Chemistry and applications of alkoxy silanes, Journal of Coatings technology, 65, 822, pages 57-60, 1993 ».

Des alcoxysilanes tels que les alkyltriéthoxysilanes et les alkyltriméthoxysilanes commercialisés sous les références Silquest A-137 (OSI Specialties) et Prosil 9202 (PCR) peuvent être utilisés pour l'enrobage des pigments.

L'utilisation des alkylpolysiloxanes ayant un groupe terminal réactif tel que alcoxy, hydroxy, halogène, amino ou imino sont décrits dans la demande JP H07-196946. Ils conviennent également pour le traitement des pigments.

*iii) Polymères de silicone-acrylate*

Des polymères de silicone-acrylique greffés ayant un squelette siliconé tels que décrits dans les brevets US 5,725,882, US 5,209,924, US 4,972,037, US 4,981,903, US 4,981,902, US 5,468,477, et dans les brevets US 5,219,560 et EP 0 388 582, peuvent être utilisés.

D'autres polymères silicone-acrylate peuvent être des polymères siliconés comportant dans leur structure le motif de formule (I) suivant :

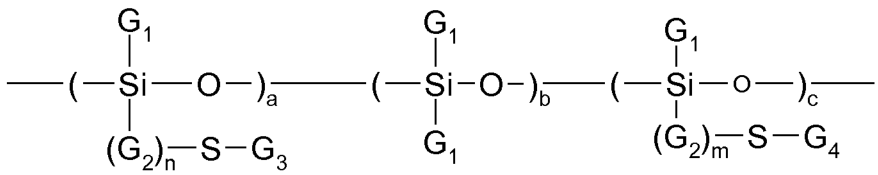

(I)

dans lequel les radicaux $G_1$, identiques ou différents, représentent l'hydrogène ou un radical alkyle en $C_1$-$C_{10}$ ou encore un radical phényle ; les radicaux $G_2$, identiques ou différents, représentent un groupe alkylène en $C_1$-$C_{10}$ ; $G_3$ représente un reste polymérique résultant de l'(homo)polymérisation d'au moins un monomère anionique à insaturation éthylénique ; $G_4$ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (I) ci-dessus présente au moins l'une, et encore plus préférentiellement l'ensemble, des caractéristiques suivantes :

- les radicaux $G_1$ désignent un radical alkyle, de préférence le radical méthyle ;
- n est non nul, et les radicaux $G_2$ représentent un radical divalent en $C_1$-$C_3$, de préférence un radical propylène ;
- $G_3$ représente un radical polymérique résultant de l'(homo)polymérisation d'au moins un monomère du type acide carboxylique à insaturation éthylénique, de préférence l'acide acrylique et/ou l'acide méthacrylique ; et
- $G_4$ représente un radical polymérique résultant de l'(homo)polymérisation d'au moins un monomère du type (méth)acrylate d'alkyle($C_1$-$C_{10}$), de préférence du type (méth)acrylate d'isobutyle ou de méthyle.

Des exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

*iv) Résines de silicone*

L'agent de surface siliconé peut être choisi parmi les résines de silicone.

Par « résine », on entend une structure tridimensionnelle.

Les résines de silicones peuvent être solubles ou gonflables dans les huiles de silicone. Ces résines sont des polymères de polyorganosiloxanes réticulés.

La nomenclature des résines de silicone est connue sous le nom de « MDTQ », la résine étant décrite en fonction des différentes unités monomères siloxane qu'elle comprend, chacune des lettres « MDTQ » caractérisant un type d'unité.

La lettre M représente l'unité monofonctionnelle de formule $(CH_3)_3SiO_{1/2}$, l'atome de silicium étant relié à un seul atome d'oxygène dans le polymère comprenant cette unité.

La lettre D signifie une unité difonctionnelle $(CH_3)_2SiO_{2/2}$ dans laquelle l'atome de silicium est relié à deux atomes d'oxygène.

La lettre T représente une unité trifonctionnelle de formule $(CH_3)SiO_{3/2}$.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tétra fonctionnelle $SiO_{4/2}$ dans laquelle l'atome de silicium est lié à quatre atomes d'hydrogène eux mêmes liés au reste du polymère.

Divers résines de propriétés différentes peuvent être obtenues à partir de ces différentes unités, les propriétés des ces polymères variant en fonction du type de monomères (ou unités), du type et du nombre de radicaux substitués, de la longueur de la chaîne polymérique, du degré de ramification et de la taille des chaînes pendantes.

A titre d'exemple de ces résines silicones, on peut citer :

- les siloxysilicates qui peuvent être des triméthylsiloxysilicate de formule $[(CH_3)_3XSiXO]_xX(SiO_{4/2})_y$ (unités MQ) dans laquelle x et y sont des entiers allant de 50 à 80,
- les polysilesquioxanes de formule $(CH_3SiO_{3/2})_{.x}$ (unités T) dans laquelle x est supérieur à 100 et dont au moins un des radicaux méthyle peut être substitué par un groupement R tel que défini plus haut,
- les polyméthylsilsesquioxanes qui sont des polysilsesquioxanes dans lesquels aucun des radicaux méthyle n'est substitué par un autre groupement. De tels polymethylsilsesquioxanes sont décrits dans le document US 5,246,694 dont le contenu est incorporé par référence.

A titre d'exemples de résines polymethylsilsesquioxanes commercialement disponibles, on peut citer celles qui sont commercialisés :

- par la société Wacker sous la référence Resin MK tels que la Belsil PMS MK : polymère comprenant des unités répétitives $CH_3SiO_{3/2}$ (unités T), pouvant aussi comprendre jusqu'à 1 % en poids d'unités $(CH_3)_2SiO_{2/2}$ (unités D) et présentant un poids moléculaire moyen d'environ 10000,
- par la société SHIN-ETSU sous les références KR-220L qui sont composé d'unités T de formule $CH_3SiO_{3/2}$ et ont des groupes terminaux Si-OH (silanol), sous la référence KR-242A qui comprennent 98 % d'unités T et 2% d'unités diméthyle D et ont des groupes terminaux Si-OH ou encore sous la référence KR-251 comprenant 88 % d'unités T et 12 % d'unités dimethyl D et ont des groupes terminaux Si-OH.

Comme résines siloxysilicates, on peut citer les résines trimethylsiloxysilicate (TMS) éventuellement sous forme de poudres. De telles résines sont commercialisées sous les références SR1000, .E. 1 170-002 ou SS 4230, par la société GENERAL ELECTRIC ou sous les références TMS 803, WACKER 803 et 804 par la société WACKER SILICONE CORPORATION.

On peut encore citer les résines timéthylsiloxysilicate commercialisées dans un solvant tel que la cyclométhicone, vendues sous la dénomination «KF-7312J» par la société Shin-Etsu, « DC 749 », « DC 593 » par la société Dow Corning.

A titre d'exemple de références commerciales de pigments traités avec un composé siliconé, on peut citer :

- l'oxyde de fer rouge/diméthicone vendu sous la référence SA-C 338075 - 10 par la société Miyoshi Kasei,
- un pigment obtenu par traitement du DC Red 7 avec un composé siliconé, commercialisé par la société Coletica sous la référence Gransil GCM (qui est un mélange D5 et de polysilicone 11).

***Agent de surface fluoré***

Les pigments peuvent être traités en surface totalement ou partiellement avec un composé de nature fluorée.

Les agents de surface fluorés peuvent être choisis parmi les phosphates de perfluoroalkyle, les perfluoropolyéthers, les polytétrafluoropolyéthylène (PTFE), les perfluoroalcanes, les perfluoroalkyl silazanes, les polyoxides d'hexafluoropropylène, les polyorganosiloxanes comprenant des groupes perfluoroalkyl perfluoropolyéthers.

On entend par radical perfluoroalkyle, un radical alkyle dans lequel tous les atomes d'hydrogène ont été remplacés par des atomes de fluor.

Les perfluoropolyéthers sont notamment décrits dans la demande de brevet EP-A-486135, et vendus sous les dénominations commerciales FOMBLIN par la société MONTEFLUOS.

Des phosphates de perfluoroalkyle sont en particulier décrits dans la demande JP H05-86984. Les phosphate-diéthanol amine de perfluoroalkyle commercialisés par Asahi Glass sous la référence AsahiGuard AG530 peuvent être utilisés.

Parmi les perfluoroalcanes linéaires, on peut citer les perfluorocycloalcanes, les perfluoro(alkylcycloalcanes), les perfluoropolycycloalcanes, les hydrocarbures perfluorés aromatiques (les perfluoroarènes) et les composés organo perfluorés hydrocarbonés comportant au moins un hétéroatome.

Parmi les perfluoroalcanes, on peut citer la série des alcanes linéaires tels que le perfluorooctane, le perfluorononane ou le perfluorodécane.

Parmi les perfluorocycloalcanes et les perfluoro(alkylcycloalcanes), on peut citer la perfluorodécaline vendue sous la dénomination de « FLUTEC PP5 GMP » par la Société RHODIA, la perfluoro(méthyldécaline), les perfluoro(C3-C5 alkyl-cyclohexanes) tels que le perfluoro(butylcyclohexane).

Parmi les perfluoropolycycloalcanes on peut citer les dérivés de bicyclo [3.3.1] nonane tel que le perfluorotriméthylbicyclo [3.3.1] nonane, les dérivés de l'adamantane tels que le perfluorodiméthyladamantane et les dérivés perfluorés de phénanthrène hydrogéné tel que le tétracosafluoro-tétradécahydrophénanthrène.

Parmi les perfluoroarènes, on peut citer les dérivés perfluorés du naphtalène comme le perfluoronaphtalène et le perfluorométhyl-1-napthtalène.

A titre d'exemple de références commerciales de pigments traités avec un composé fluoré, on peut citer :

- L'oxyde de fer jaune/phosphate de perfluoroalkyle vendu sous la référence PF 5 Yellow 601 par la société Daito Kasei,
- L'oxyde de fer rouge/phosphate de perfluoroalkyle vendu sous la référence PF 5 Red R 516L par la société Daito Kasei,
- L'oxyde de fer noir/phosphate de perfluoroalkyle vendu sous la référence PF 5 Black BL 100 par la société Daito Kasei,
- Le dioxyde de titane/phosphate de perfluoroalkyle vendu sous la référence PF 5 TiO2 CR 50 par la société Daito Kasei,
- L'oxyde de fer jaune/perfluoropolymethylisopropylether vendu sous la référence Iron oxyde yellow BF-25-3 par la société Toshiki,
- Le DC Red 7/perfluoropolymethylisopropylether vendu sous la référence D&C Red 7 FHC par la société Cardre Inc.,
- Le DC Red 6/PTFE vendu sous la référence T 9506 par la société Warner - Jenkinson.

***Agent de surface fluoro-siliconé***

Les pigments peuvent être traités en surface totalement ou partiellement avec un composé de nature fluoro-siliconée.

Le composé fluoro-siliconé peut être choisi parmi les perfluoroalkyl diméthicones, les perfluoroalkyl silanes et les perfluoroalkyl trialcoxysilanes.

On peut citer comme perfluoroalkyl silanes, les produits LP-IT et LP-4T commercialisés par Shin-Etsu Silicone.

Les perfluoroalkyl diméthicones peuvent être représentés par la formule suivante:

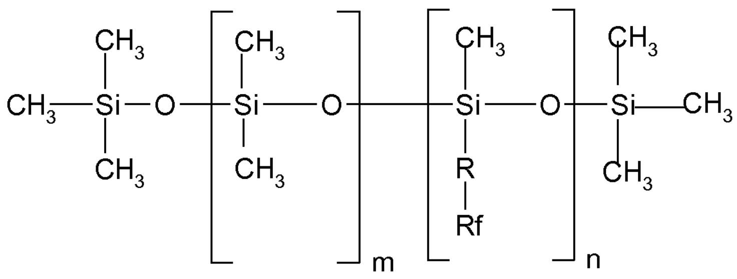

dans laquelle :

- R représente un groupement divalent alkyle linéaire ou ramifié, ayant 1 à 6 atomes de carbone, de préférence un groupement divalent méthyle, éthyle, propyle ou butyle,
- Rf représente un radical perfluoroalkyle, ayant 1 à 9 atomes de carbone, de préférence 1 à 4 atomes de carbone,
- m est choisi entre 0 à 150, de préférence de 20 à 100, et
- n est choisi entre 1 à 300, de préférence de 1 à 100.

A titre d'exemple de références commerciales de pigment traité avec un composé fluoro-siliconé, on peut citer le dioxyde de titane/fluorosilicone vendu sous la référence Fluorosil Titanium dioxyde 100TA par la société Advanced Dermaceuticals International Inc.

***Autres agents de surface lipophiles***

L'agent de traitement hydrophobe peut également être choisi parmi :

- les savons métalliques comme le dimyristate d'aluminium, le sel d'aluminium du glutamate de suif hydrogéné ;

A titre de savons métalliques, on peut notamment citer les savons métalliques d'acides gras ayant de 12 à 22 atomes de carbone, et en particulier ceux ayant de 12 à 18 atomes de carbone.
Le métal du savon métallique peut notamment être du zinc ou du magnésium. Comme savon métallique, on peut utiliser le laurate de zinc, le stéarate de magnésium, le myristate de magnésium, le stéarate de zinc, et leurs mélanges.
L'acide gras peut notamment être choisi parmi l'acide laurique, l'acide myristique, l'acide stéarique, l'acide palmitique.

- les acides aminés N-acylés ou leurs sels qui peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle.

L'acide aminé peut être par exemple la lysine, l'acide glutamique ou l'alanine.
Les sels de ces composés peuvent être les sels d'aluminium, de magnésium, de calcium, de zirconium, de zinc, de sodium, de potassium.
Ainsi, selon un mode de réalisation particulièrement préféré, un d'acide aminé N-acylé peut être notamment un dérivé d'acide glutamique et/ou un de ses sels, et plus particulièrement un stéaroyl glutamate, comme par exemple le stéaroyl glutamate d'aluminium.

- la lécithine et ses dérivés,
- le triisostéaryle titanate d'isopropyle ,

A titre d'exemples de pigments traités par l'isopropyl titanium triisostéarate (ITT), on peut citer ceux vendus sous la référence commerciale BWBO-I2 (Iron oxyde CI77499 and isopropyl titanium triisostéarate), BWYO-I2 (Iron oxyde CI77492 and isopropyl titanium triisostéarate), et BWRO-I2 (Iron oxyde CI77491 and isopropyl titanium triisostéarate) par la société KOBO.

- le sébaçate d'isostéaryle,
- les cires naturelles végétales ou animales ou les cires synthétiques polaires,
- les esters gras, en particulier par des esters de jojoba ;
- les phospholipides ; et leurs mélanges.

Les cires mentionnées dans les composés cités précédemment peuvent être celles utilisées généralement dans le domaine cosmétique, telles que définies par la suite.
Elles peuvent notamment être hydrocarbonées, siliconées et/ou fluorées, comportant éventuellement des fonctions ester ou hydroxyle. Elles peuvent être également d'origine naturelle ou synthétique.
On entend par cire polaire une cire contenant des composés chimiques comportant au moins un groupement polaire. Les groupements polaires sont bien connus de l'homme du métier ; il peut s'agir par exemple de groupement alcool, ester, acide carboxylique. Ne font pas partie des cires polaires les cires de polyéthylène, les cires de paraffine, les cires microcristallines, l'ozokérite, les cires de Fisher-Tropsch.

En particulier, les cires polaires ont un paramètre moyen de solubilité dA de HANSEN à 25 °C tel que dA > 0 $(J/cm^3)^{1/2}$ et mieux dA > 1 $(J/cm^3)^{1/2}$.

$$\delta_a = \sqrt{\delta_p^2 + \delta_h^2}$$

où dP et dH sont respectivement les contributions polaires et de types interactions spécifiques aux paramètres de solubilité de Hansen.

La définition des solvants dans l'espace de solubilité tridimensionnel selon HANSEN est décrite dans l'article de C. M. HANSEN : « The three dimensionnal solubility parameters » J. Paint Technol. 39, 105 (1967) ;

- dH caractérise les forces d'interactions spécifiques (type liaisons hydrogène, acide/base, donneur/accepteur, etc) ;
- dP caractérise les forces d'interactions de DEBYE entre dipôles permanents ainsi que les forces d'interactions de KEESOM entre dipôles induits et dipôles permanents.

Les paramètres dP et dH sont exprimés en $(J/cm^3)½$.

Une cire polaire est notamment constituée de molécules comportant, outre des atomes de carbone et d'hydrogène dans leur structure chimique, des hétéroatomes (tels que O, N, P).

A titre illustratif et non limitatif de ces cires polaires, on peut notamment citer les cires polaires naturelles, comme la cire d'abeille, la cire de lanoline, la cire d'orange, la cire de citron, et les cires d'insectes de Chine ; la cire de son de riz, la cire de Carnauba, la cire de Candellila, la cire d'Ouricury, la cire de fibres de liège, la cire de canne à sucre, la cire du Japon et la cire de sumac; la cire de montan.

Les pigments enrobés selon l'invention par au moins un composé lipophile, peuvent être présents dans une composition de l'invention en une teneur allant de 0,1 à 30 % en poids, par rapport au poids total de la composition, mieux de 1 à 15 % en poids.

Selon un mode de réalisation particulier, les pigments peuvent être enrobés par au moins un composé choisi parmi les agents de surface siliconés ; les agents de surface fluorés ; les acides aminés N-acylés ou leurs sels ; le trisostéaryle titanate d'isopropyle ; les cires naturelles végétales ou animales ; les esters gras ; et leurs mélanges.

Selon un mode de réalisation particulièrement préféré, les pigments peuvent être enrobés par un acide aminé N-acylé et/ou un de ses sels, en particulier par un dérivé d'acide glutamique et/ou un de ses sels, notamment un stearoyl glutamate, comme par exemple, le stearoyl glutamate d'aluminium, ou par un ester gras, en particulier par un ester de jojoba.

A titre d'exemples de pigments enrobés selon l'invention, on peut citer plus particulièrement les composés suivants :

- Oxyde de fer enrobé enrobé de stearoyl glutamate, par exemple commercialisé par la société MIYOSHI sous la référence « NAI-C33-7001-10 »
- Oxyde de fer enrobé par des esters de jojoba, par exemple commercialisé par la société KOBO sous la référence « BWBO-NJE2 »; et
- Dioxyde de titane et oxyde de fer, enrobés de stéaroyl glutamate d'aluminium, par exemple commercialisé sous la référence NAI par MIYOSHI KASEI.

**<u>Charges</u>**

La composition selon l'invention peut également comprendre au moins une charge.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, la silice traitée en surface par un agent hydrophobe, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la Société Dow Corning) et les microbilles de résine de silicone (Tospearlse® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

On peut également utiliser un composé susceptible de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel® 820 DU 40 et Expancel®007WU par la Société AKZO NOBEL.
Les charges peuvent représenter de 0,1 à 25 %, en particulier de 0,2 à 20 % en poids par rapport au poids total de la composition.

Fibres

Les compositions conformes à l'invention peuvent également comprendre au moins une fibre, permettant notamment une amélioration de l'effet allongeant.
Par « fibre », il faut comprendre un objet de longueur L et de diamètre D tel que L soit supérieur à D, et de préférence, très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.
Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.
En particulier, les fibres ont une longueur allant de 1 μm à 10 mm, de préférence de 0,1 mm à 5 mm et mieux de 0,3 mm à 3 mm. Leur section peut être comprise dans un cercle de diamètre allant de 2 nm à 500 μm, de préférence allant de 100 nm à 100 μm et mieux de 1 μm à 50 μm. Le poids ou titre des fibres est souvent donné en denier ou décitex et représente le poids en gramme pour 9 km de fil. De préférence, les fibres selon l'invention ont un titre choisi dans la gamme allant de 0,01 à 10 deniers, de préférence de 0,1 à 2 deniers et mieux de 0,3 à 0,7 deniers.
Les fibres utilisables dans les compositions selon l'invention peuvent être choisies parmi les fibres rigides ou non rigides, elles peuvent être d'origine synthétique ou naturelle, minérales ou organiques.
Par ailleurs, les fibres peuvent être traitées ou non en surface, enrobées ou non, colorées ou non colorées.
A titre de fibres utilisables dans les compositions selon l'invention, on peut citer les fibres non rigides telles que les fibres de polyamide (Nylon®) ou les fibres rigides telles que les fibres de polyimide-amide comme celles vendues sous les dénominations KERMEL®, KERMEL TECH® par la société RHODIA ou de poly-(p-phénylène-téréphtalamide) (ou d'aramide) notamment vendues sous la dénomination Kevlar® par la société DUPONT DE NEMOURS.
Les fibres peuvent êtres présentes en une teneur allant de 0,01 % à 10 % en poids, par rapport au poids total de la composition, en particulier de 0,1 % à 5 % en poids, et plus particulièrement de 0,3 à 3 % en poids.
Une composition de l'invention peut en outre comprendre tout actif cosmétique tel que les actifs choisis parmi les antioxydants, les conservateurs, les parfums, les actifs bactéricides ou anti-transpirants, les neutralisants, les émollients, les hydratants, les épaississants, les agents de coalescence, les plastifiants, les vitamines, des filtres en particulier solaires, et leurs mélanges.
Bien entendu, l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.
Selon un mode de réalisation préféré, la composition selon l'invention est non rincée.
La composition selon l'invention peut être conditionnée dans un récipient délimitant au moins un compartiment qui comprend ladite composition, ledit récipient étant fermé par un élément de fermeture.
Le récipient est de préférence associé à un applicateur, notamment sous forme d'une brosse comportant un arrangement de poils maintenus par un fil torsadé. Une telle brosse torsadée est décrite notamment dans le brevet US 4,887,622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple dans le brevet FR 2 761 959. Avantageusement, l'applicateur est solidaire d'une tige qui, elle même, est solidaire de l'élément de fermeture.
L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par « encliquetage » on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.
Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou

alliage).

Le récipient est de préférence équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

L'invention est illustrée plus en détails dans les exemples suivants qui sont présentés à titre illustratif et non limitatif de l'invention.

## EXEMPLES

### Exemple 1

| | Composés/ références commerciales | % massique |
|---|---|---|
| A | Oxyde de fer noir | 4 |
| | Gomme de xanthane | 0,6 |
| | Laponite (silicate mixte magnésium/ lithium/ sodium) | 0,6 |
| | Eau désionisée | 58,8 |
| | Alcool éthylique absolu dénaturé | 8 |
| | Sel mono-sodique de n-stéaroyl-L-acide glutamique (vendu sous la référence Amisoft HS 11 par la société AJINOMOTO) | 1 |
| B | Cire d'abeille organique désodorisée | 22 |
| | Mélange de cétylstéaryl glucoside et d'alcools cétylique, stéarylique (20/80) (vendu sous la référence MONTANOV 68 ® par la société SEPPIC) | 5 |
| **TOTAL** | | 100 |

**Mode opératoire** : Les agents épaississants, le tensioactif glutamate et les colorants sont dispersés sous agitation dans la phase aqueuse. La phase A ainsi formée est ensuite portée à 90 °C. Puis la phase A est versée dans la phase grasse B contenant les cires et le tensioactif glucoside à 90 °C. L'émulsion est réalisée par agitation à l'aide d'un agitateur Moritz.

On refroidit ensuite l'émulsion jusqu'à 60 °C sous agitation rotor-stator, puis sous agitation lente à l'aide d'une pâle plate jusqu'à 25 °C.

L'émulsion cire-dans-eau ainsi obtenue présente un pH de 6,7, et une viscosité mesurée le jour suivant sa préparation, à 25 °C, avec un appareil Rheomat 180 de la société Lamy équipé d'un mobile MS-R4, après 10 minutes de rotation à une vitesse de 200 t/min$^{-1}$, de 5,4 Pa.s.

### Exemple 2

| | Composés/ références commerciales | % massique |
|---|---|---|
| A | Oxyde de fer noir | 7 |
| | Gomme de xanthane | 0,2 |
| | Gomme arabique | 3 |
| | Glycérol | 3 |
| | Déhydroacétate de sodium | 0,5 |
| | Eau désionisée | 52,1 |
| | Sel mono-sodique de n-stéaroyl-L-acide glutamique vendu sous la référence Amisoft HS 11 par la société AJINOMOTO | 1 |

(suite)

| | Composés/ références commerciales | % massique |
|---|---|---|
| B | Cire d'abeille organique désodorisée | 14 |
| | Cire organique de Carnauba | 8 |
| | Mélange de cétylstéaryl glucoside et d'alcools cétylique, stéarylique (20/80) (vendu sous la référence MONTANOV 68 ® par la société SEPPIC) | 3 |
| | Mono/distéarate de glycéryle | 3 |
| C | Acide sorbique | 0,2 |
| | Alcool éthylique absolu dénaturé 96 degrés | 5 |
| TOTAL | | 100 |

La phase C constituée du mélange d'acide sorbique et d'alcool éthylique est ajoutée au mélange des phases A et B.

La composition est préparée comme indiqué en exemple 1. Sa viscosité mesurée le jour suivant selon le protocole décrit en exemple 1 est de 7,6 Pa.s.

Exemple 3

| | Composés/ références commerciales | % massique |
|---|---|---|
| A | Oxyde de fer noir | 5 |
| | Gomme de xanthane | 0,2 |
| | Gomme arabique | 3 |
| | Glycérol | 3,8 |
| | Déhydroacétate de sodium | 0,5 |
| | Eau désionisée | 51,3 |
| | Sel mono-sodique de n-stéaroyl-L-acide glutamique vendu sous la référence Amisoft HS 11 par la société AJINOMOTO | 1 |
| B | Cire d'abeille organique désodorisée | 14 |
| | Cire organique de Carnauba | 8 |
| | Mélange de cétylstéaryl glucoside et d'alcools cétylique, stéarylique (20/80) (vendu sous la référence MONTANOV 68 ®par la société SEPPIC) | 5 |
| | Mono/distéarate de glycéryle | 3 |
| C | Acide sorbique | 0,2 |
| | Alcool éthylique absolu dénaturé 96 degrés | 5 |
| **TOTAL** | | 100 |

La composition est préparée comme indiqué en exemples 1 et 2. Sa viscosité mesurée le jour suivant selon le protocole décrit en exemple 1 est de 8,3 Pa.s.

Exemple 4

| | Composés/ références commerciales | % massique |
|---|---|---|
| A | Sel mono-sodique de n-stéaroyl-L-acide glutamique vendu sous la référence Amisoft HS 11 par la société AJINOMOTO | 0,7 |
| | Gomme de xanthane | 0,6 |
| | Gomme arabique | 3 |
| | Glycérol | 3 |
| | Déhydroacétate de sodium | 0,5 |
| | Eau désionisée | 56,6 |
| B | Oxyde de fer noir enrobé de stearoyl glutamate d'aluminium (3 %)[a] | 5 |
| | Cire de Candelilla | 3 |
| | Beurre de karité | 1 |
| | Huile pure végétale d'Argan | 0,1 |
| | Cire d'abeille organique désodorisée | 8,7 |
| | Cire organique de Carnauba | 6,3 |
| | Mélange de cétylstéaryl glucoside et d'alcools cétylique, stéarylique (20/80) (vendu sous la référence MONTANOV 68 ®par la société SEPPIC) | 2,1 |
| | Mono/distéarate de glycéryle | 4,2 |
| C | Acide sorbique | 0,2 |
| | Alcool éthylique absolu dénaturé 96 degrés | 5 |
| TOTAL | | 100 |
| [a] NAI-C33-7001-10 de Miyoshi | | |

La composition est préparée comme indiqué en exemples 1 et 2. Sa viscosité mesurée le jour suivant selon le protocole décrit en exemple 1 est de 8,5 Pa.s.

L'ensemble des compositions des exemples 1 à 4 présentent un pouvoir chargeant satisfaisant, permettant d'obtenir un maquillage épais des cils.

Exemple 5

Cet exemple vise à démontrer la synergie obtenue par la présence simultanée d'un dérivé d'acide glutamique et/ou un de ses sels et d'un alkylpolyglycoside.

| | A (comparatif) | B (invention) | C (comparatif) |
|---|---|---|---|
| Composition | 5 % MONTANOV 68®(SEPPIC) | 4 % MONTANOV 68® (SEPPIC) | |
| | | 1 % Glutamate[b] | 5 % Glutamate[b] |
| | 30 % Phytowax olive 18L57 (SOPHIM) | 30 % Phytowax olive 18L57 (SOPHIM) | 30 % Phytowax olive 18L57 SOPHIM |
| | 0,6 % Gomme de xanthane | 0,6 % Gomme de xanthane | 0,6 % Gomme de xanthane |
| | 0,6 % Lithium Magnésium Sodium Silicate | 0,6 % Lithium Magnésium Sodium Silicate | 0,6 % Lithium Magnésium Sodium Silicate |
| | QS eau | QS eau | QS eau |

(suite)

| Résultats | | | |
|---|---|---|---|
| **Aspect macroscopique** | Pâte papier mâché, pas lisse | Belle crème, épaisse, cireuse, brillante et lisse | Crème épaisse, brillante et lisse |
| **Commentaire sur l'observation microscopique** | Dispersion très grossière des cires | Dispersion des cires et des gels. Dispersion homogène | Présence d'amas de gels. Dispersion hétérogène |
| (b) Amisoft HS 11 par la société AJINOMOTO | | | |

Cet essai comparatif démontre l'effet avantageux obtenu par la présence simultanée d'un dérivé d'acide glutamique et/ou un de ses sels et d'un alkylpolyglycoside. Ainsi, on observe que cette association permet l'obtention d'une dispersion à la fois fine et homogène des cires.

**Revendications**

1. Composition cosmétique de revêtement des fibres kératiniques, en particulier des cils, **caractérisée en ce qu'**elle se présente sous la forme d'une émulsion cire-dans-eau et **en ce qu'**elle comprend l'association d'au moins un dérivé d'acide glutamique et/ou un de ses sels et d'au moins un alkylpolyglycoside, la ou lesdites cires étant distincte(s) des alcools gras comprenant de 10 à 30 atomes de carbone,
ledit dérivé d'acide glutamique étant le sel mono-sodique de n-stéaroyl-L-acide glutamique et ledit alkylpolyglycoside étant le cétylstéaryl glucoside, notamment mis en oeuvre en mélange avec l'alcool cétylstéarylique.

2. Composition selon la revendication précédente, **caractérisée en ce que** ledit sel-monosodique d'acide glutamique est présent en une teneur allant de 0,1 à 2 % en poids, de préférence de 0,2 à 1 % en poids par rapport au poids total de la composition.

3. Composition selon l'une quelconque des revendications précédentes, **caractérisée en ce que** ledit alkylpolyglycoside est présent en une teneur allant de 0,2 à 2 % en poids, de préférence de 0,3 à 1,6 % en poids par rapport au poids total de la composition.

4. Composition selon l'une quelconque des revendications précédentes, **caractérisée en ce que** ledit alkylpoly glycoside et ledit sel mono-sodique d'acide glutamique sont mis en oeuvre dans un rapport pondéral alkylpolyglycoside/dérivé d'acide glutamique, supérieur ou égal à 0,2, avantageusement supérieur ou égal à 0,3, et plus particulièrement inférieur à 2, avantageusement inférieur à 1,6.

5. Composition selon l'une quelconque des revendications précédentes, **caractérisée en ce qu'**elle comprend au moins 15 % en poids en cires, de préférence au moins 18 % en poids et plus préférentiellement, au moins 20 % en poids en cires par rapport au poids total de la composition.

6. Composition selon l'une quelconque des revendications précédentes, **caractérisée en ce qu'**elle comprend moins de 1 %, de préférence moins de 0,5 % en poids de triéthanolamine ou l'un de ses dérivés, et mieux, est exempte de triéthanolamine ou de ses dérivés.

7. Composition cosmétique selon l'une quelconque des revendications précédentes **caractérisée en ce qu'**elle comprend au moins un additif choisi parmi les composés pâteux, les polymères filmogènes, les gélifiants, les huiles, les matières colorantes, les charges, les fibres, les antioxydants, les conservateurs, les parfums, les actifs bactéricides ou anti-transpirants, les neutralisants, les émollients, les épaississants, les agents de coalescence, les plastifiants, les hydratants, les vitamines et les filtres en particulier les filtres solaires, et leurs mélanges.

8. Composition selon l'une quelconque des revendications précédentes, **caractérisée en ce qu'**elle comprend au moins un pigment enrobé, notamment par au moins un composé lipophile, par exemple choisi parmi les agents de surface siliconés ; les agents de surface fluorés ; les agents de surface fluoro-siliconés ; les savons métalliques , les acides aminés N-acylés ou leurs sels ; la lécithine et ses dérivés ; le trisostéaryle titanate d'isopropyle ; le sébaçate d'isostéaryle; les cires naturelles végétales ou animales; les cires synthétiques polaires ; les esters gras ;

les phospholipides, et leurs mélanges.

9. Procédé cosmétique de maquillage et/ou de soin non thérapeutique des fibres kératiniques, notamment des cils, comprenant l'application sur lesdites fibres kératiniques d'une composition selon l'une quelconque des revendications précédentes.

10. Utilisation d'une composition selon l'une quelconque des revendications 1 à 8, pour obtenir sur les fibres kératiniques, en particulier sur les cils, un maquillage chargeant.

## Patentansprüche

1. Kosmetische Zusammensetzung zum Überziehen der Keratinfasern, insbesondere der Wimpern, **dadurch gekennzeichnet, dass** sie in der Form einer Wachs-in-Wasser-Emulsion vorliegt und dass sie die Kombination von mindestens einem Glutaminsäurederivat und/oder einem ihrer Salze und mindestens ein Alkylpolyglycosid umfasst, wobei das oder die Wachse verschieden sind von Fettalkoholen, die 10 bis 30 Kohlenstoffatome umfassen, wobei das Glutaminsäurederivat das Mononatriumsalz von n-Stearoyl-L-glutaminsäure ist und das Alkylpolyglycosid Cetylstearylglucosid ist, das insbesondere im Gemisch mit Cetylstearylalkohol eingesetzt wird.

2. Zusammensetzung nach dem vorangehenden Anspruch, **dadurch gekennzeichnet, dass** das Mononatriumsalz von Glutaminsäure in einem Gehalt von 0,1 bis 2 Gew.-%. vorzugsweise von 0,2 Gew.-% bis 1 Gew.-% bezogen auf das Gesamtgewicht der Zusammensetzung vorhanden ist.

3. Zusammensetzung nach einem der vorangehenden Ansprüche, **dadurch gekennzeichnet, dass** das Alkylpolyglycosid in einem Gehalt von 0,2 bis 2 Gew.-%. vorzugsweise von 0,3 bis 1,6 Gew.-% bezogen auf das Gesamtgewicht der Zusammensetzung vorhanden ist.

4. Zusammensetzung nach einem der vorangehenden Ansprüche, **dadurch gekennzeichnet, dass** das Alkylpolyglycosid und das Mononatriumsalz von Glutaminsäure in einem Gewichtsverhältnis von Alkylpolyglycosid/Glutaminsäurederivat von größer oder gleich 0,2, zweckmäßigerweise von größer oder gleich 0,3 und spezieller kleiner als 2, zweckmäßigerweise kleiner als 1,6 eingesetzt werden.

5. Zusammensetzung nach einem der vorangehenden Ansprüche, **dadurch gekennzeichnet, dass** sie mindestens 15 Gew.-% Wachse, vorzugsweise mindestens 18 Gew.-% und stärker bevorzugt mindestens 20 Gew.-% Wachse bezogen auf das Gesamtgewicht der Zusammensetzung umfasst.

6. Zusammensetzung nach einem der vorangehenden Ansprüche, **dadurch gekennzeichnet, dass** sie mindestens 1 Gew.-%, vorzugsweise weniger als 0,5 Gew.-% Triethanolamin oder eines seiner Derivate umfasst, und besser noch frei ist von Triethanolamin oder von seinen Derivaten.

7. Kosmetische Zusammensetzung nach einem der vorangehenden Ansprüche, **dadurch gekennzeichnet, dass** sie mindestens ein Additiv umfasst, das ausgewählt ist aus pastösen Verbindungen, filmbildenden Polymeren, Gelbildnern, Ölen, Farbstoffen, Füllstoffen, Fasern, Antioxidantien, Konservierungsstoffen, Duftstoffen, bakteriziden oder antitranspirativen Wirkstoffen, Neutralisationsmitteln, Weichmachern, Verdickungsmitteln, Koaleszenzmitteln, Plastifizierern, Hydratationsmitteln, Vitaminen und Filtermitteln, insbesondere Sonnenfiltern und ihren Gemischten.

8. Zusammensetzung nach einem der vorangehenden Ansprüche, **dadurch gekennzeichnet, dass** sie mindestens ein, insbesondere durch mindestens eine lipophile Verbindung, umhülltes Pigment umfasst, die beispielsweise ausgewählt ist aus siliconierten oberflächenaktiven Mitteln, fluorierten oberflächenaktiven Mitteln, fluoro-siliconierten oberflächenaktiven Mitteln, Metallseifen, N-acylierten Aminosäuren oder ihren Salzen; Lecithin und seinen Derivaten; Isopropyltrisostearyltitanat, Isostearylsebacat, natürlichen pflanzlichen oder tierischen Wachsen, polaren synthetischen Wachsen, Fettestern, Phospholipiden, und ihren Gemischen.

9. Kosmetisches nicht therapeutisches Schmink- oder Pflegeverfahren der Keratinfasern, insbesondere der Wimpern, umfassend das Aufbringen auf die Keratinfasern einer Zusammensetzung nach einem der vorangehenden Ansprüche.

10. Verwendung einer Zusammensetzung nach einem Ansprüche 1 bis 8 zum Erhalt auf den Keratinfasern, insbesondere

auf den Wimpern, einer Schrninkauftragung.

## Claims

1. A cosmetic composition for coating keratin fibres, particularly eyelashes, **characterized in that** it appears as a wax-in-water emulsion and **in that** it comprises the combination of at least one glutamic acid derivative and/or salt thereof and at least one alkylpolyglycoside, said wax or waxes being distinct from fatty alcohols comprising from 10 to 30 carbon atoms,
said glutamic acid derivative being the monosodium salt of n-stearoyl-L-glutamic acid, and said alkylpolyglycoside being cetylstearyl glucoside, notably applied in a mixture with cetylstearyl alcohol.

2. The composition according to the preceding claim, **characterized in that** said monosodium salt of glutamic acid is present in a content ranging from 0.1 to 2% by weight, preferably from 0.2 to 1% by weight based on the total weight of the composition.

3. The composition according to any of the preceding claims, **characterized in that** said alkylpolyglycoside is present in a content ranging from 0.2 to 2% by weight, preferably from 0.3 to 1.6% by weight based on the total weight of the composition

4. The composition according to any of the preceding claims, **characterized in that** said alkylpolyglycoside and said monosodium salt of glutamic acid are applied in a weight alkylpolyglycoside/glutamic acid derivative ratio, greater than or equal to 0.2, advantageously greater than or equal to 0.3, and more particularly less than 2, advantageously less than 1.6.

5. The composition according to any of the preceding claims, **characterized in that** it comprises at least 15% by weight of waxes, preferably at least 18% by weight and more preferentially at least 20% by weight of waxes based on the total weight of the composition.

6. The composition according to any of the preceding claims, **characterized in that** it comprises less than 1%, preferably less 0.5% by weight of triethanolamine or of one of its derivatives, and better is free of triethanolamine or its derivatives.

7. The cosmetic composition according to any of the preceding claims, **characterized in that** it comprises at least one additive selected from pasty compounds, film-forming polymers, gelling agents, oils, coloring agents, fillers, fibers, antioxidants, preservatives, perfumes, bactericidal or antiperspirant actives, neutralizing agents, emollients, thickeners, coalescents, plasticizers, moisturizers, vitamins and screening agents, particularly sunscreen agents, and mixtures thereof.

8. The composition according to any of the preceding claims, **characterized in that** it comprises at least one pigment which is coated, especially with at least one lipophilic compound, selected for example from silicone surface agents; fluorinated surface agents; fluorosilicone surface agents; metal soaps, N-acylamino acids or salts thereof; lecithin and derivatives thereof; isopropyl triisostearyltitanate; isostearyl sebacate; natural plant or animal waxes; synthetic polar waxes; fatty esters; phospholipids; and mixtures thereof.

9. A cosmetic method for makeup and/or non-therapeutic care of keratinous fibers, notably eyelashes, comprising the application to said keratinous fibers of a composition according to any of the preceding claims.

10. The use of a composition according to any of claims 1 to 8 in order to obtain charging makeup on keratinous fibres, particularly the eyelashes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 02056940 A
- EP 0773017 A
- WO 9206778 A
- WO 9513863 A
- WO 9847610 A
- FR 2792190 A
- FR 2232303 A
- US 5162410 A
- WO 2004073626 A
- US 5874069 A
- US 5919441 A
- US 6051216 A
- US 5981680 A
- EP 1411069 A
- WO 04028488 A
- WO 04055081 A
- EP 847752 A
- FR 2679771
- EP 1184426 A
- US 6225198 B
- US 5990479 A
- US 4578266 A
- JP H07196946 B
- US 5725882 A
- US 5209924 A
- US 4972037 A
- US 4981903 A
- US 4981902 A
- US 5468477 A
- US 5219560 A
- EP 0388582 A
- US 5246694 A
- EP 486135 A
- JP H0586984 B
- US 4887622 A
- FR 2796529
- FR 2761959
- FR 2792618


### Littérature non-brevet citée dans la description


- Microemulsions Theory and Practice. Academic Press, 1977, 21-32
- Encyclopedia of Chemical Technology, KIRK-OTHMER. WILEY, 1979, vol. 22, 333-432
- **DABBOUSSI B.O. et al.** CdSe)ZnS core-shell quantum dots : synthesis and characterisation of a size series of highly luminescent nanocristallites. *Journal of phisical chemistry B,* 1997, vol. 101, 9463-9475
- **PENG, XIAOGANG et al.** Epitaxial Growth of highly Luminescent CdSe/CdS core/shell nanocrystals with photostability and electronic accessibility. *Journal of the American Chemical Society,* vol. 119 (30), 7019-7029
- **WITUCKI.** *A silane primer, Chemistry and applications of alkoxy silanes, Journal of Coatings technology,* 1993, vol. 65 (822), 57-60
- **C. M. HANSEN.** The three dimensionnal solubility parameters. *J. Paint Technol.,* 1967, vol. 39, 105